# EUROPEAN PATENT APPLICATION

(11) **EP 3 744 399 A1**
(43) Date of publication of application: **02.12.2020**
(21) Application number: 19305668.6
(22) Date of filing: 27.05.2019
(51) Int. Cl.: A61P 35/00, A61K 31/444, C07F 15/00

(54) **RUTHENIUM (II) COMPLEXES AND THEIR USE AS ANTICANCER AGENTS**

(71) Applicant: Paris Sciences et Lettres - Quartier Latin, 75006 Paris (FR); Ecole Nationale Superieure de Chimie Paris, 75231 Paris Cedex 05 (FR); UNIVERSITY OF ZURICH, 8006 Zürich (CH)
(72) Inventor: GASSER, Gilles Albert, 75013 PARIS (FR); FREI, Angelo, 9220 BISCHOFSZELL (CH); RUBBIANI, Ricardo, 54 78467 KONSTANZ (DE); NOTARO, Anna, 75015 PARIS (FR)
(74) Representative: Regimbeau

(57) **Abstract**

The present invention relates to a compound of the following formula (I): 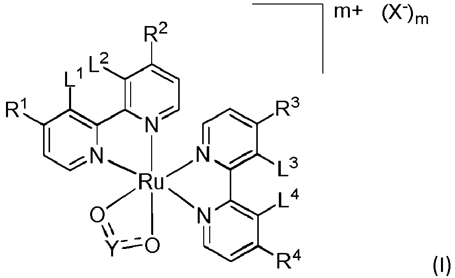 or a pharmaceutically acceptable salt and/or solvate thereof, notably for use as a drug, in particular in the treatment of cancer.

The present invention also relates to a pharmaceutical composition comprising such a compound and to a method for the preparation of such a compound.

## Description

### Field of the invention

The present invention relates to ruthenium (II) complexes bearing polypyridyl ligands and a dioxolane ligand, their uses as drug, in particular in the treatment of cancer, and their synthesis.

### Background of the invention

In the last decades, the search for new chemotherapeutic agents against cancer has challenged scientists worldwide. Chemotherapy, together with surgery, radiotherapy and immunotherapy, is used in the so-called combination therapy to treat cancer which is considered as one of the most efficient treatments (Urruticoechea, A. *et al*., 2010). Cisplatin is one of the most common chemotherapeutic agents used against cancer. However, its severe side effects (nephrotoxicity, neurotoxicity and ototoxicity) are limiting its clinical use (Rosenberg, B. *et al*., 1965). To try to address this problem, metal complexes other than platinum complexes have been examined (Rosenberg, B. *et al.,* 1967, Rosenberg, B. *et al.,* 1969 and Rosenberg, B., 1971). In this field, ruthenium complexes are playing a central role due to their advantages (i.e. multiple stable oxidation states, higher selectivity towards cancer cells, etc.) (Pieter, P., Lippard, S.J., 1997 and Wong, E., Giandomenico, C.M., 1999). KP-1019, KP-1339 and NAMI-A are, to date, the only three Ru complexes to have reached clinical trial as anticancer agents. Their mechanism of action involves ligand exchange, resembling therefore the one of cisplatin (Lebwohl, D., Canetta, R., 1998).

There exists still a need for new ruthenium complexes useful as chemotherapeutic agent having limited side effects.

### Summary of the invention

The inventors have thus designed new ruthenium (Ru) polypyridyl complexes bearing a dioxolane ligand with a great potential in the treatment of cancer.

In a first aspect, the present invention thus relates to a compound of the following formula (I): 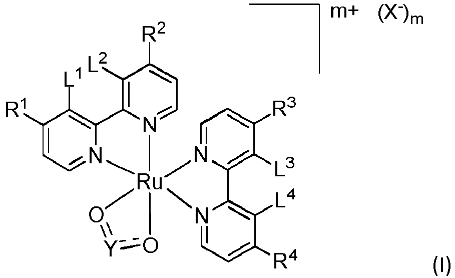 wherein
L¹ and L² are each independently selected in the group consisting of H, halogen, optionally substituted C₁-C₆ alkyl, optionally substituted C₂-C₆ alkenyl, optionally substituted C₂-C₆ alkynyl, optionally substituted carbocycle, optionally substituted aryl, optionally substituted heteroaryl, optionally substituted heterocycle, CN, NO₂, COR¹¹, OR¹² and NR¹³R¹⁴, or
L¹ and L² together represent 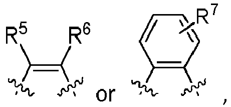
L³ and L⁴ are each independently selected in the group consisting of H, halogen, optionally substituted C₁-C₆ alkyl, optionally substituted C₂-C₆ alkenyl, optionally substituted C₂-C₆ alkynyl, optionally substituted carbocycle, optionally substituted aryl, optionally substituted heteroaryl, optionally substituted heterocycle, CN, NO₂, COR¹⁵, OR¹⁶ and NR¹⁷R¹⁸ or
L³ and L² together represent 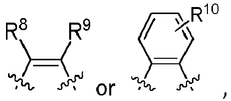
R¹ to R⁶, R⁸ and R⁹ are each independently selected in the group consisting of H, halogen, optionally substituted C₁-C₆ alkyl, optionally substituted C₂-C₆ alkenyl, optionally substituted C₂-C₆ alkynyl, optionally substituted carbocycle, optionally substituted aryl, optionally substituted heteroaryl, optionally substituted heterocycle, CN, NO₂, COR¹⁹, OR²⁰ and NR²¹R²²,
R⁷ and R¹⁰ each independently represent one or several substituent(s) independently selected in the group consisting of H, halogen, optionally substituted C₁-C₆ alkyl, optionally substituted C₂-C₆ alkenyl, optionally substituted C₂-C₆ alkynyl, optionally substituted carbocycle, optionally substituted aryl, optionally substituted heteroaryl, optionally substituted heterocycle, CN, NO₂, COR²³, OR²⁴ and NR²⁵R²⁶,
R¹¹, R¹⁵, R¹⁹ and R²³ are each independently selected in the group consisting of H, optionally substituted C₁-C₆ alkyl, OR²⁷ and NR²⁸R²⁹,
R¹², R¹³, R¹⁴, R¹⁶, R¹⁷, R¹⁸, R²⁰, R²¹, R²², R²⁴, R²⁵, R²⁶, R²⁷, R²⁸ and R²⁹ are each independently selected in the group consisting of H, optionally substituted C₁-C₆ alkyl and optionally substituted CO-C₁-C₆alkyl,
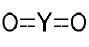 is a bidentate ligand, selected in the group consisting of: 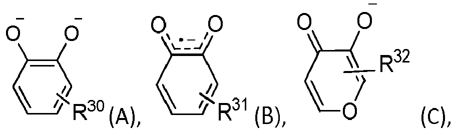
in which
R³⁰, R³¹ and R³² each independently represent one or several substituent(s) independently selected in the group consisting of H, halogen, optionally substituted C₁-C₆ alkyl, CN, NO₂, COR³³, OR³⁴ and NR³⁵R³⁶, R³³ is H, halogen, optionally substituted C₁-C₆ alkyl, OR³⁷ or NR³⁸R³⁹,
R³⁴ to R³⁹ are each independently selected in the group consisting of H, optionally substituted C₁-C₆ alkyl and optionally substituted CO-C₁-C₆alkyl,
X⁻ is a pharmaceutically acceptable anion, preferably selected in the group consisting of PF₆⁻, Cl⁻, Br⁻, BF₄⁻, (C₁-C₆ alkyl)-C(O)O⁻, (C₁-C₆ haloalkyl)-C(O)O⁻, (C₁-C₆ alkyl)-SO₃⁻and (C₁-C₆ haloalkyl)-SO₃⁻,
m is the global charge of the ruthenium complex of formula: 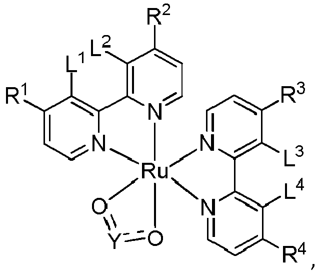 and
m is equal to 0 when 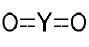 is A and m is equal to +1 when 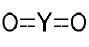 is B or C,
or a pharmaceutically acceptable salt and/or solvate thereof.

Preferably, according to this first aspect, the compound of formula (I) or a pharmaceutically acceptable salt and/or solvate thereof is not one of the following compounds:
- bis(2,2'-bipyridine)(3,5-bis(1,1-dimethylethyl)-1,2-benzenediolato)ruthenium (II) monohydrate;
- bis(2,2'-bipyridine)(1,2-benzenediolato)ruthenium (II) sesquihydrate;
- bis(2,2'-bipyridine)(3,4,5,6-tetrachloro-1,2-benzenediolato)ruthenium (II) hemihydrate;
- bis(2,2'-bipyridine)(3,5-bis(1,1-dimethylethyl)-3,5-cyclohexadiene-1,2-dionato(radical(1-))ruthenium (II) perchlorate diethyl ether solvate;
- bis(2,2'-bipyridine)(3,5-bis(1,1-dimethylethyl)-3,5-cyclohexadiene-1,2-dionato(radical(1-))ruthenium (II) hexafluorophosphate.

These compounds are disclosed in Haga *et al.* 1986. No biological activity is reported in this article for these compounds.

In a second aspect, the present invention relates to a pharmaceutical composition comprising at least one compound of formula (I) and at least one pharmaceutically acceptable excipient.

In a third aspect, the present invention relates to a compound of formula (I) according to the invention or a pharmaceutically acceptable salt and/or solvate thereof, or a pharmaceutical composition according to the present invention for use as a drug, in particular in the treatment of cancer.

The present invention also relates to the use of a compound of formula (I) according to the invention or a pharmaceutically acceptable salt and/or solvate thereof, or a pharmaceutical composition according to the present invention for the manufacture of a drug, notably intended in the treatment of cancer.

The present invention also relates to the use of a compound of formula (I) according to the invention or a pharmaceutically acceptable salt and/or solvate thereof, or a pharmaceutical composition according to the present invention for the treatment of cancer.

The present invention also relates to a method for the treatment of cancer comprising the administration to a person in need thereof of an effective dose of a compound of formula (I) according to the invention or a pharmaceutically acceptable salt and/or solvate thereof, or a pharmaceutical composition according to the present invention.

In a fourth aspect, the present invention concerns a method of preparation of compounds of formula (I) according to the invention.

### Definitions

The term "stereoisomers" used in this invention refers to configurational stereoisomers and more particularly to optical isomers.

In the present invention, the optical isomers result in particular from the different position in space of the three bidentate ligands of the ruthenium. Ruthenium thus represents a chiral or asymmetric center. Optical isomers that are not mirror images of one another are thus designated as "diastereoisomers", and optical isomers, which are non-superimposable mirror images are designated as "enantiomers".

An equimolar mixture of two enantiomers of a chiral compound is designated as a racemic mixture or racemate.

For the purpose of the invention, the term "pharmaceutically acceptable" is intended to mean what is useful to the preparation of a pharmaceutical composition, and what is generally safe and non-toxic, for a pharmaceutical use.

The term "pharmaceutically acceptable salt and/or solvate" is intended to mean, in the framework of the present invention, a salt and/or solvate of a compound which is pharmaceutically acceptable, as defined above, and which possesses the pharmacological activity of the corresponding compound. The pharmaceutically acceptable salts comprise:
(1) acid addition salts formed with inorganic acids such as hydrochloric, hydrobromic, sulfuric, nitric and phosphoric acid and the like; or formed with organic acids such as acetic, benzenesulfonic, fumaric, glucoheptonic, gluconic, glutamic, glycolic, hydroxynaphtoic, 2-hydroxyethanesulfonic, lactic, maleic, malic, mandelic, methanesulfonic, muconic, 2-naphtalenesulfonic, propionic, succinic, dibenzoyl-L25 tartaric, tartaric, p-toluenesulfonic, trimethylacetic, and trifluoroacetic acid and the like, and
(2) base addition salts formed when an acid proton present in the compound is either replaced by a metal ion, such as an alkali metal ion, an alkaline-earth metal ion, or an aluminium ion; or coordinated with an organic or inorganic base. Acceptable organic bases comprise diethanolamine, ethanolamine, N-methylglucamine, triethanolamine, tromethamine and the like. Acceptable inorganic bases comprise aluminium hydroxide, calcium hydroxide, potassium hydroxide, sodium carbonate and sodium hydroxide.

Acceptable solvates for the therapeutic use of the compounds of the present invention include conventional solvates such as those formed during the last step of the preparation of the compounds of the invention due to the presence of solvents. As an example, mention may be made of solvates due to the presence of water (these solvates are also called hydrates) or ethanol.

The term "halogen", as used in the present invention, refers to a fluorine, bromine, chlorine or iodine atom.

The term "C₁-C₆ alkyl", as used in the present invention, refers to a straight or branched monovalent saturated hydrocarbon chain containing from 1 to 6 carbon atoms including, but not limited to, methyl, ethyl, n-propyl, iso-propyl, n-butyl, iso-butyl, sec-butyl, *t*-butyl, n-pentyl, n-hexyl, and the like.

The term "C₂-C₆ alkenyl", as used in the present invention, refers to a straight or branched monovalent unsaturated hydrocarbon chain containing from 2 to 6 carbon atoms and comprising at least one double bond including, but not limited to, ethenyl, propenyl, butenyl, pentenyl, hexenyl and the like. The term "C₂-C₆ alkynyl", as used in the present invention, refers to a straight or branched monovalent unsaturated hydrocarbon chain containing from 2 to 6 carbon atoms and comprising at least one triple bond including, but not limited to, ethynyl, propynyl, propynyl, butynyl, pentynyl, hexynyl and the like. The term "C₁-C₆ alkoxy", as used in the present invention, refers to a C₁-C₆ alkyl chain as defined above linked to the rest of the molecule via an oxygen atom including, but not limited to, methoxy, ethoxy, n-propoxy, iso-propoxy, n-butoxy, iso-butoxy, sec-butoxy, t-butoxy, n-pentoxy, n-hexoxy, and the like. In particular, it can be a methoxy.

The term "C₁-C₆ haloalkyl" refers to a C₁-C₆ alkyl chain as defined above wherein one or more hydrogen atoms are replaced by a halogen atom selected from fluorine, chlorine, bromine or iodine, preferably a fluorine atom. For example, it is a CF₃ group.

The term "carbocycle" refers to a non-aromatic hydrocarbon ring, saturated or unsaturated, typically comprising from 3 to 20 carbons and comprising one or more fused or bridged ring(s). In particular, it is a saturated hydrocarbon cycle, especially a C₃-C₇ cycloalkyl, including, but not limited to, cyclopropyl, cyclobutyl, cyclopentyl, cyclohexyl, cycloheptyl, and the like.

The term "C₃-C₇ cycloalkyl" refers to a saturated hydrocarbon ring comprising from 3 to 7 carbons, including cyclopropyl, cyclobutyl, cyclopentyl, cyclohexyl and cycloheptyl.

The term "heterocycle" as used in the present invention refers to a non-aromatic, saturated or unsaturated monocycle or polycycle (comprising fused, bridged or spiro rings) comprising preferably 5 to 10, notably 5 or 6, atoms in the ring(s), in which the atoms of the ring(s) consist of carbon atoms and one or more, advantageously 1 to 4, and more advantageously 1 or 2, heteroatoms, such as a nitrogen, oxygen or sulphur atom, the remainder being carbon atoms. A heterocycle can be notably piperidinyl, piperizinyl, pyrrolidinyl, pyrazolidinyl, imidazolidinyl, azepanyl, thiazolidinyl, isothiazolidinyl, oxazocanyl, thiazepanyl, benzimidazolonyl.

When the heterocycle is substituted, it is advantageously substituted by a group selected in the group consisting of C₁-C₆ alkyl and oxo.

The term "aryl" refers to an aromatic hydrocarbon group preferably comprising from 6 to 12 carbon atoms and comprising one or more fused rings, such as, for example, a phenyl or naphthyl group. Advantageously, it is a phenyl group.

The term "heteroaryl", as used in the present invention, refers to an aromatic group comprising one or several, notably one or two, fused hydrocarbon cycles in which one or several, notably one to four, advantageously one or two, carbon atoms each have been replaced with a heteroatom selected from a sulfur atom, an oxygen atom and a nitrogen atom, preferably selected from an oxygen atom and a nitrogen atom. It can be a furyl, thienyl, pyrrolyl, pyridyl, oxazolyl, isoxazolyl, thiazolyle, isothiazolyl, imidazolyl, pyrazolyl, oxadiazolyl, thiadiazolyl, triazolyl, tetrazolyl, pyridazinyl, pyrimidinyl, pyrazinyl, triazinyl, quinolyl, isoquinolyl, quinoxalyl or indyl.

In the context of the present invention, "unsaturated" means that the hydrocarbon chain may contain one or more unsaturation(s), i.e. a double bond C=C, advantageously one.

In the context of the present invention, "optionally substituted" means that the group in question is optionally substituted with one or more substituents which may be selected in particular from halogen, C₁-C₆ alkyl, C₁-C₆ haloalkyl, aryl, oxo, NR^{a}R^{b}, COR^{c}, CO₂R^{d}, CONR^{e}R^{f}, OR^{g}, CN and NO₂ wherein R^{a} to R^{g} are, independently of one another, H, C₁-C₆ alkyl, C₁-C₆ haloalkyl or aryl, preferably H or C₁-C₆ alkyl. The term "pharmaceutical composition" is meant in the framework of the present invention a composition having preventive and curative properties towards cancers.

The term "catecholate ligand" refers to the following ligand which is optionally substituted with one or more substituent(s) R³⁰ as defined here: 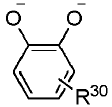 wherein each oxygen atom is linked to the ruthenium of the compound of the invention.

The term "semiquinonate ligand" refers to the following oxidized form of the catecholate ligand which is optionally substituted with one or more substituent(s) R³¹ as defined here: 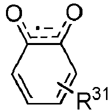 wherein each oxygen atom is linked to the ruthenium of the compound of the invention. The semiquinonate ligand corresponds to a radical anion. 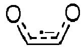 means that the radical is delocalized on both oxygen and the three bonds between said oxygen atoms. It corresponds to an equilibrium between the different following mesomeric forms:

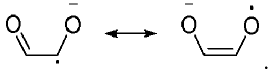

The term "Electron-withdrawing group" (EWG) refers to an atom or a chemical group able to remove electron density to neighboring atoms from itself, usually by resonance or inductive effect. EWG includes, but are not limited to, NO₂, CN, halogen, in particular fluorine, C₁-C₆ haloalkyl, such as CF₃, CBr₃ or CCl₃, haloformyl group such as COCl, COBr and COI, CORⁱ, C(O)ORⁱⁱ and CONRⁱⁱⁱR^{iv} with Rⁱ, Rⁱⁱ, Rⁱⁱⁱ and R^{iv} each independently representing H, C₁-C₆ alkyl or C₁-C₆ haloalkyl, such as CHO, CO-C₁-C₆ alkyl, COOH, C(O)O-C₁-C₆ alkyl, CONH₂ or CONH-C₁-C₆alkyl.

The term "Electron-donating group" (EDG) (also called electron-releasing group) refers to an atom or a chemical group able to release electron density to neighboring atoms from itself, usually by resonance or inductive effect. EDG includes, but are not limited to, C₁-C₆ alkyl, OR^{m}, NRⁿR^{o}, OC(O)R^{p} and NR^{q}C(O)R^{r}, with R^{m}, Rⁿ, R^{o}, R^{p}, R^{q} and R^{r} each independently representing H or C₁-C₆ alkyl, such as OH, C₁-C₆ alkoxy group, NH₂, NH-C₁-C₆alkyl, OC(O)- C₁-C₆ alkyl or NHC(O)- C₁-C₆ alkyl.

The term "antimetastatic agent" refers to a pharmaceutical agent which prevents or impairs tumoral cells metastasis, i.e. the spread or the migration of said tumoral cells from the organ in which they first appeared to another part of the body.

### Detailed description

### Compound of formula I

According to a particular embodiment, the compound of the present invention may correspond to the following formulas:

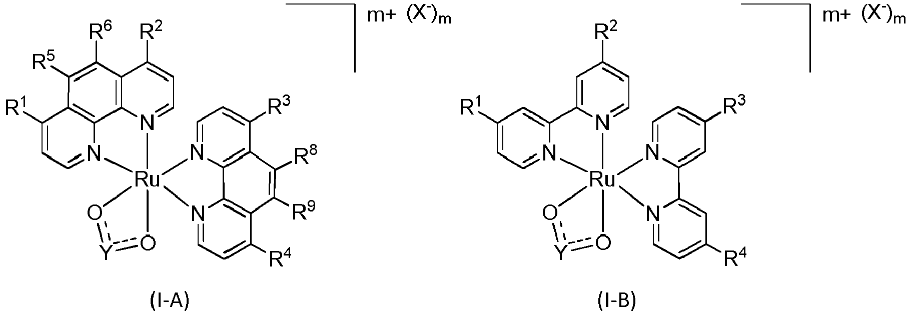

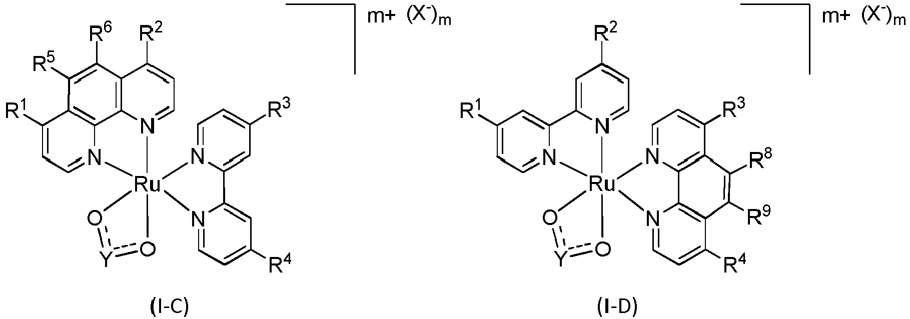

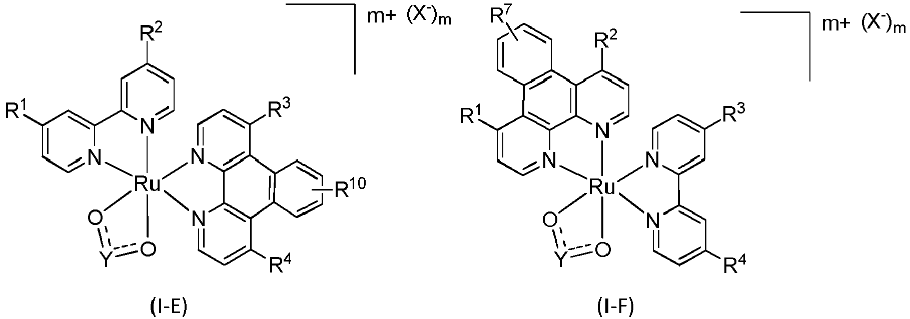

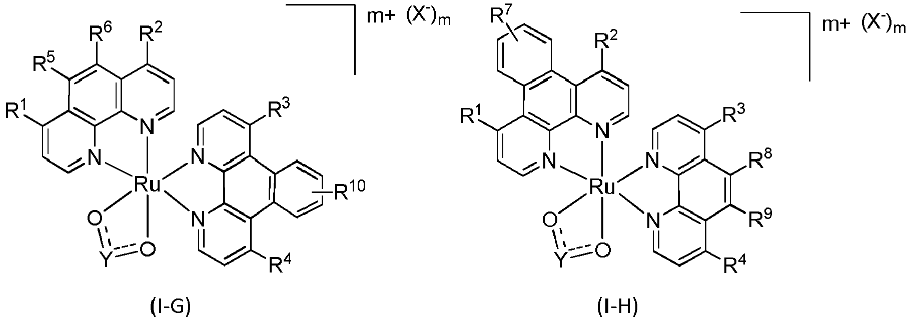

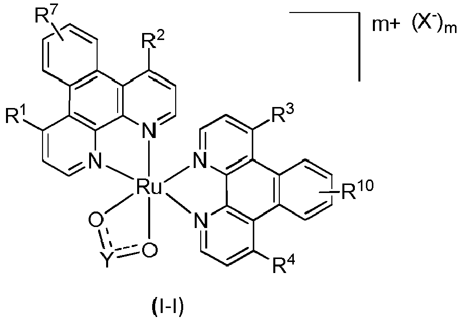

Preferably, it is a compound of formula (IA), (I-B) or (I-I), in particular (I-A).

The compounds according to the present invention can be in the form of a stereoisomer or a mixture of stereoisomers, such as a mixture of enantiomers, notably a racemic mixture.

Advantageously, in compounds of formula (I), L¹ and L² are each independently selected in the group consisting of H, halogen, optionally substituted C₁-C₆ alkyl, optionally substituted C₂-C₆ alkenyl, optionally substituted C₂-C₆ alkynyl, optionally substituted aryl, CN, NO₂, COR¹¹, OR¹² and NR¹³R¹⁴, or L¹ and L² together represent 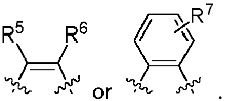 Preferably, L¹ and L² are each independently selected in the group consisting of H, halogen, C₁-C₆ alkyl, aryl, COR¹¹, OR¹² and NR¹³R¹⁴, or L¹ and L² together represent

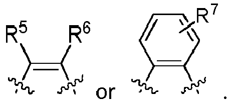

Advantageously, in compounds of formula (I), L³ and L⁴ are each independently selected in the group consisting of H, halogen, optionally substituted C₁-C₆ alkyl, optionally substituted C₂-C₆ alkenyl, optionally substituted C₂-C₆ alkynyl, optionally substituted aryl, CN, NO₂, COR¹¹, OR¹² and NR¹³R¹⁴, or L³ and L² together represent 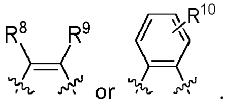 Preferably, L³ and L⁴ are each independently selected in the group consisting of H, halogen, C₁-C₆ alkyl, aryl, COR¹⁵, OR¹⁶ and NR¹⁷R¹⁸ or L³ and L⁴ together represent

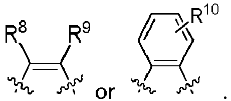

In the previous embodiments, R⁵, R⁶, R⁸ and R⁹ are preferably each independently selected in the group consisting of H, halogen, optionally substituted C₁-C₆ alkyl, optionally substituted aryl, optionally substituted heteroaryl, optionally substituted heterocycle, CN, NO₂, COR¹⁹, OR²⁰ and NR²¹R²². More preferably, R⁵, R⁶, R⁸ and R⁹ are each independently selected in the group consisting of H, halogen, OR²⁰, NR²¹R²², optionally substituted C₁-C₆ alkyl and optionally substituted aryl; preferably selected in the group consisting of H, halogen, OR²⁰, NR²¹R²², C₁-C₆ alkyl and aryl with R²⁰, R²¹ and R²² each independently being H or C₁-C₆ alkyl; notably preferably selected in the group consisting of H, halogen, C₁-C₆ alkyl and aryl.

In the previous embodiments, R⁷ and R¹⁰ each independently represent advantageously one or several substituent(s), in particular 1 or 2 substituents, independently selected in the group consisting of H, halogen, optionally substituted C₁-C₆ alkyl, optionally substituted aryl, optionally substituted heteroaryl, optionally substituted heterocycle, CN, NO₂, COR²³, OR²⁴ and NR²⁵R²⁶. More preferably, R⁷ and R¹⁰ each independently represent one or several substituent(s), in particular 1 or 2 substituents, especially one substituent independently selected in the group consisting of H, halogen, OR²⁴, NR²⁵R²⁶, optionally substituted C₁-C₆ alkyl and optionally substituted aryl; preferably selected in the group consisting of H, halogen, OR²⁴, NR²⁵R²⁶, C₁-C₆ alkyl and aryl with R²⁴, R²⁵ and R²⁶ each independently being H or C₁-C₆ alkyl; notably preferably selected in the group consisting of H, halogen, C₁-C₆ alkyl and aryl.

In a preferred embodiment, L¹ and L² may each be H or L¹ and L² together represent 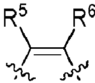 or 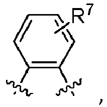 wherein R⁵ to R⁷ are as defined above. Preferably, L¹ and L² together represent 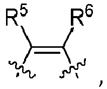 wherein R⁵ and R⁶ are as defined above and advantageously are independently H, halogen, OR²⁰, NR²¹R²², optionally substituted C₁-C₆ alkyl or optionally substituted aryl. More preferably, R⁵ and R⁶ are independently H, halogen, OR²⁰, NR²¹R²², C₁-C₆ alkyl or aryl, preferably with R²⁰, R²¹ and R²² each independently being H or C₁-C₆ alkyl; notably are independently H, halogen, C₁-C₆ alkyl or aryl. In a preferred embodiment, R⁵ and R⁶ are identical. Even more preferably, R⁵ and R⁶ are H.

In a preferred embodiment, L³ and L⁴ may each be H or L³ and L⁴ together represent 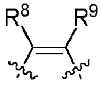 or 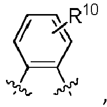 wherein R⁸ to R¹⁰ are as defined above. Preferably, L³ and L⁴ together represent 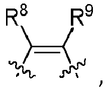 wherein R⁸ and R⁹ are as defined above and advantageously are independently H, halogen, OR²⁰, NR²¹R²², optionally substituted C₁-C₆ alkyl or optionally substituted aryl. More preferably, R⁸ and R⁹ are independently H, halogen, OR²⁰, NR²¹R²², C₁-C₆ alkyl or aryl, preferably with R²⁰, R²¹ and R²² each independently being H or C₁-C₆ alkyl; notably are independently H, halogen, C₁-C₆ alkyl or aryl. In a preferred embodiment, R⁸ and R⁹ are identical. Even more preferably, R⁵ and R⁶ are H.

According to a preferred embodiment, L¹ and L³ are identical and L² and L⁴ are identical.

In a particular embodiment, L¹ to L⁴ may be identical. For example, L¹ to L⁴ are H and the compound of the invention is a compound of formula (I-B).

In the compound of formula (I) or (I-A) to (I-I) of the invention, R¹ to R⁴ are preferably each independently selected in the group consisting of H, halogen, optionally substituted C₁-C₆ alkyl and optionally substituted aryl, such as H, halogen, C₁-C₆ alkyl and aryl. More preferably, R¹ to R⁴ are each independently an optionally substituted aryl, such as an aryl, for example, but not limited to, a phenyl or a naphtyl. Even more preferably, R¹ to R⁴ are identical. Typically, R¹ to R⁴ are phenyl.

In a particular embodiment, the compound of formula (I) is a compound of formula (I-A) wherein R¹ to R⁴ are phenyl and R⁵, R⁶, R⁸ and R⁹ are H, halogen or OH, preferably H.

The present invention advantageously relates to compound of formula (I), wherein 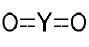 is a bidendate ligand selected in the group consisting of: 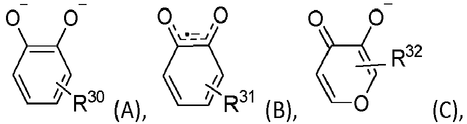 in which
R³⁰, R³¹ and R³² each independently represent one or several substituent(s), notably 1, 2, 3 or 4 substituent(s), independently selected in the group consisting of H, halogen, optionally substituted C₁-C₆ alkyl, NO₂, COR³³ and OR³⁴, in particular H, halogen, C₁-C₆ alkyl, NO₂ and OR³⁴,
R³³ is H, halogen, optionally substituted C₁-C₆ alkyl, OR³⁷ or NR³⁸R³⁹,
R³⁴ to R³⁹ are each independently selected in the group consisting of H, optionally substituted C₁-C₆ alkyl and optionally substituted CO-C₁-C₆alkyl, preferably H and optionally substituted C₁-C₆ alkyl.

Preferably, R³⁴ is C₁-C₆ alkyl, such as methyl.

Ligand A corresponds to a catecholate ligand which carries two negative charges. Then, when 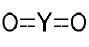 is under catecholate form A, the compound of formula (I) is neutral and corresponds to the following formula 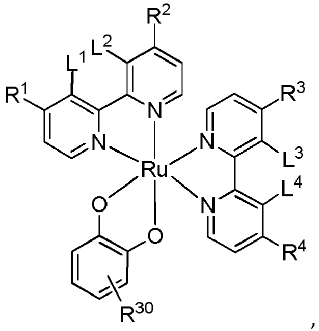 m being equal to 0. R³⁰ preferably represents one or several substituent(s), notably 1, 2, 3 or 4 substituent(s), in particular 1 or 4 substituent(s), independently selected in the group consisting of H, halogen, optionally substituted C₁-C₆ alkyl, CN, NO₂ and COR³³, wherein R³³ is as defined above. More preferably, R³⁰ represent one or several substituent(s), notably 1, 2, 3 or 4 substituent(s), selected in the group consisting of halogen, NO₂ and C₁-C₆ haloalkyl. Even more preferably, R³⁰ represent one or several substituent(s), notably 1, 2, 3 or 4 substituent(s), in particular 1 or 4 substituent(s), selected in the group consisting of halogen, such as bromine, and NO₂.

Ligand B corresponds to a semiquinonate ligand which carries one negative charge. Then, when 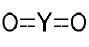 is under semiquinonate form B, the compound of formula (I) is positively charged and corresponds to the following formula 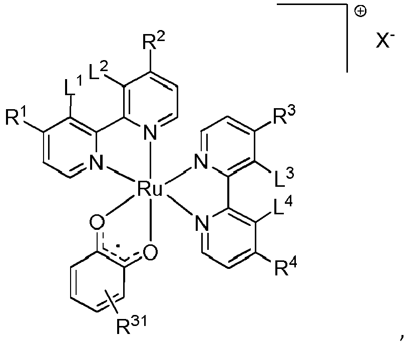 m being equal to +1. R³¹ preferably represents one or several substituent(s), notably 1, 2, 3 or 4 substituent(s), in particular 1 or 2 substituent(s), independently selected in the group consisting of H, optionally substituted C₁-C₆ alkyl, OR³⁴ and NR³⁵R³⁶, wherein R³⁴, R³⁵ and R³⁶ are as defined above, and preferably H or C₁-C₆ alkyl. More preferably, R³¹ represent one or several substituent(s), notably 1, 2, 3 or 4 substituent(s), in particular 1 or 2 substituent(s), independently selected in the group consisting of H, C₁-C₆ alkyl and OR³⁴, R³⁴ being advantageously H or a C₁-C₆ alkyl such as a methyl.

Ligand C is pyromeconic acid (3-Hydroxy-4H-pyran-4-one) derivative ligand which also carries one negative charge. Then, when 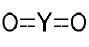 is form C, the compound of formula (I) is positively charged and corresponds to the following formula: 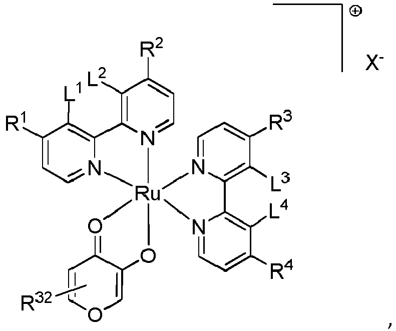 m being equal to +1.

R³² preferably represents one or several substituent(s), notably 1, 2 or 3 substituent(s), in particular 1 substituent, independently selected in the group consisting of H, optionally substituted C₁-C₆ alkyl, OR³⁴ and NR³⁵R³⁶, wherein R³⁴, R³⁵and R³⁶ are as defined above, in particular H, C₁-C₆ alkyl and OR³⁴, R³⁴ being advantageously H or a C₁-C₆ alkyl such as a methyl. More preferably, R³² represent one substituent selected in the group consisting of H and C₁-C₆ alkyl such as a methyl.

The present invention preferably relates to compound of formula (I) wherein 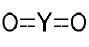 is under form B wherein R³¹ is as described above.

X⁻ is a pharmaceutically acceptable anion selected so as to obtain an overall electrically neutral compound of formula (I), preferably selected among PF₆⁻, Cl⁻, Br⁻, BF₄⁻, (C₁-C₆ alkyl)-C(O)O⁻, (C₁-C₆ haloalkyl)-C(O)O⁻, (C₁-C₆ alkyl)- SO₃⁻ and (C₁-C₆ haloalkyl)-SO₃⁻, more preferably it is PF₆⁻, Cl⁻, Br⁻, BF₄⁻, CH₃C(O)O⁻, CF₃C(O)O⁻ and CF₃SO₃⁻. Even more preferably, X⁻ is PF₆⁻.

In a particular embodiment, the compound of formula (I) is a compound of formula (I-A) wherein R¹ to R⁴ are phenyl, R⁵, R⁶, R⁸ and R⁹ are H, halogen or OH, preferably H, and 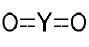 is under form B wherein R³¹ preferably represents one or several H, C₁-C₆ alkyl such as methyl or tert-butyl, or a methoxy group.

According to a preferred embodiment, the present invention relates to the following compounds of formula (I):

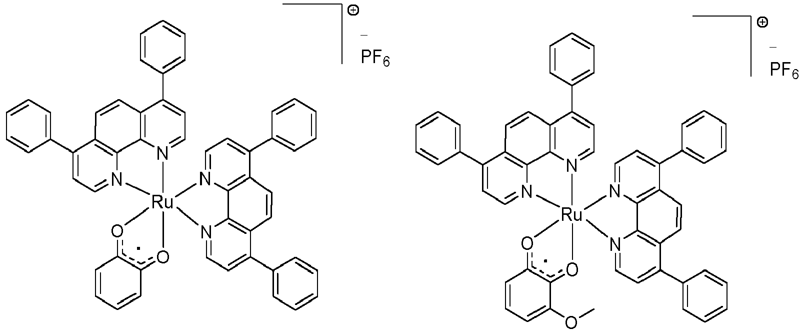

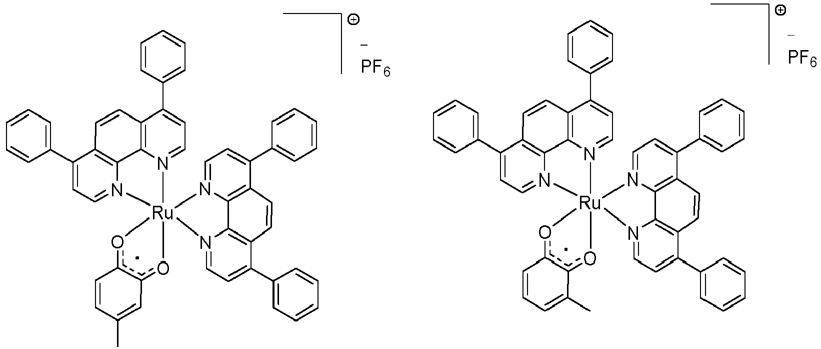

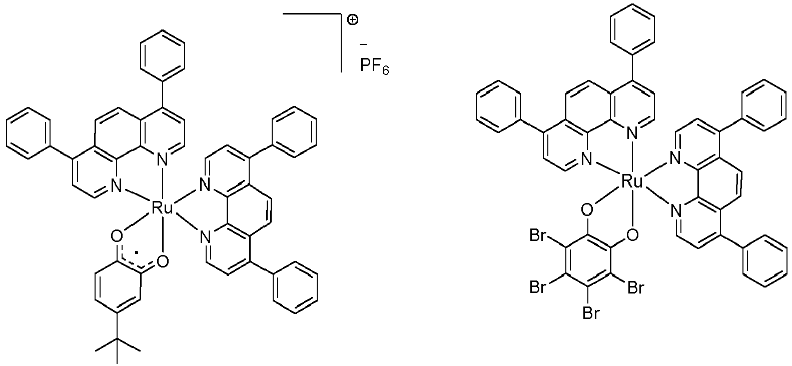

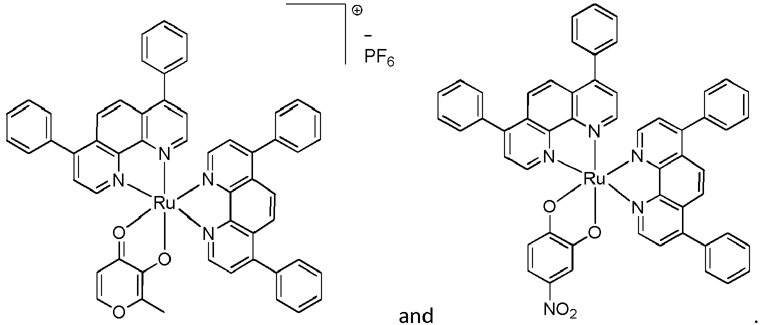

### Pharmaceutical composition

The present invention also relates to a pharmaceutical composition comprising at least one compound of formula (I) or a pharmaceutically acceptable salt and/or solvate thereof, and at least one pharmaceutically acceptable excipient.

The pharmaceutical compositions of the invention can be intended to oral or parenteral (e.g. subcutaneous, intramuscular, intravenous) administration, preferably oral or intravenous administration. The active ingredient can be administered in unit forms for administration, mixed with conventional pharmaceutical carriers, to animals, preferably mammals including humans.

For oral administration, the pharmaceutical composition can be in a solid or liquid (solution or suspension) form.

A solid composition can be in the form of tablets, gelatin capsules, powders, granules and the like. In tablets, the active ingredient can be mixed with pharmaceutical vehicle(s) such as gelatin, starch, lactose, magnesium stearate, talc, gum arabic and the like before being compressed. The tablets may be further coated, notably with sucrose or with other suitable materials, or they may be treated in such a way that they have a prolonged or delayed activity. In powders or granules, the active ingredient can be mixed or granulated with dispersing agents, wetting agents or suspending agents and with flavor correctors or sweeteners. In gelatin capsules, the active ingredient can be introduced into soft or hard gelatin capsules in the form of a powder or granules such as mentioned previously or in the form of a liquid composition such as mentioned below.

A liquid composition can contain the active ingredient together with a sweetener, a taste enhancer or a suitable coloring agent in a solvent such as water. The liquid composition can also be obtained by suspending or dissolving a powder or granules, as mentioned above, in a liquid such as water, juice, milk, etc. It can be for example a syrup or an elixir.

For parenteral administration, the composition can be in the form of an aqueous suspension or solution which may contain suspending agents and/or wetting agents. The composition is advantageously sterile. It can be in the form of an isotonic solution (in particular in comparison to blood).

The compounds of the invention can be used in a pharmaceutical composition at a dose ranging from 0.01 mg to 1000 mg a day, administered in only one dose once a day or in several doses along the day, for example twice a day in equal doses. The daily administered dose is advantageously comprised between 5 mg and 500 mg, and more advantageously between 10 mg and 200 mg. However, it can be necessary to use doses out of these ranges, which could be noticed by the person skilled in the art.

### Treatment

The compound of formula (I) or a pharmaceutically acceptable salt and/or solvate thereof, or the pharmaceutical composition according to the present invention are useful as a drug, notably in the treatment of cancer. In particular, the compound of formula (I) or a pharmaceutically acceptable salt and/or solvate thereof, or the pharmaceutical composition according to the present invention can be used as antimetastatic agent.

According to a particular embodiment, cancer may be selected from lung cancer (e.g non-small cell lung cancer), ovarian cancer, colon cancer, colorectal cancer, stomach cancer, testicular cancer, head cancer, neck cancer, breast cancer, kidney cancer, liver cancer, skin cancer, tongue cancer, pancreatic cancer, gall bladder cancer, bladder cancer, glioblastoma, neuroblastoma or leukemia (e.g. acute myeloid leukemia, myelodysplastic syndrome or chronic myelomonocytic leukemia).

### Method of preparation

The present invention also relates to a method of preparation of compound of formula (I) or pharmaceutically acceptable salt and/orsolvate thereof, said method comprising the following steps:
(i) reacting a compound of formula (II) 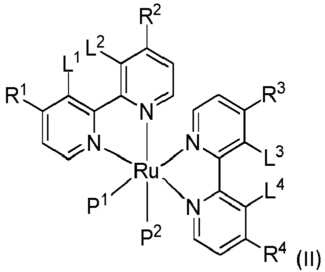
   in which L¹ to L⁴ and R¹ to R⁴ are as defined above,
   P¹ and P² each independently represent halogen, OR⁴⁰ or S(O)R⁴¹R⁴²,
   R⁴⁰ is H or C₁-C₆ alkyl,
   R⁴¹ and R⁴² each independently represent a C₁-C₆ alkyl, such as a methyl,
   with a compound of formula (III) 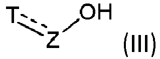 in which 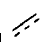 represents a single or a double bond, T is O when 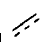 is a double bond and T is OH when 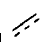 is a single bond and Z is selected in the group consisting of: 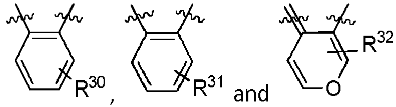 in which R³⁰, R³¹ and R³² are as defined in claim 1. in the presence of a base such as NaOH under inert atmosphere,
(ii) placing the reaction mixture of step (i) in contact with an oxidant, such as O₂,
(iii) if necessary, reacting the product resulting from step (ii) with a salt A⁺X⁻, wherein X⁻ is as defined above and A⁺ is a counter cation preferably selected from H⁺, Na⁺, K⁺, Li⁺ and N⁺R'R"R"'R"", with R', R", R'" and R"" independently being H or C₁-C₆ alkyl.

### Step (i)

Compound of formula (II) can be obtained following methods described in the literature.

In a preferred embodiment, in compound of formula (II), P¹ and P² are identical and are preferably both halogen, in particular Cl. Therefore, compound of formula (II) wherein P¹ and P² are Cl can be obtained, for example, starting from commercially available complex Ru(DMSO)₄(Cl)₂ by reaction with suitable phenantroline derivative according to a method described in the literature (Caspar, R. et al., 2006).

Compound of formula (III) is commercially available or it can be obtained starting from catechol or pyromeconic acid by functionalization reactions well-known to the skilled person in the art.

Step (i) corresponds to a ligand exchange wherein the P¹ and P² are replaced by the bidentate ligand 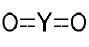 as described in compound of formula (I) thanks to the reaction with a compound of formula (III).

During step (i), a compound of formula (II) reacts with a base, in particular a strong base such as NaOH, KOH, Ca(OH)₂, Ba(OH)₂, NaH, KH or LiOH, in particular NaOH, and a compound of formula (III). The reaction is preferably carried out in a polar solvent, preferably selected among alcohols, such as methanol, ethanol, propanol, butanol. Preferably, the solvent is propanol, notably, 2-propanol.

The reaction is carried out under inert atmosphere such as nitrogen (N₂) or argon (Ar) atmosphere. The reaction is preferably carried out at a temperature corresponding to the boiling temperature of the solvent.

Step (i) is advantageously achieved in dark. Indeed, it is suitable that the reaction is protected from light to avoid side reactions.

According to one embodiment, in compound of formula (III), T is OH and 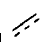 is a single bond. Compound of formula (III) thus corresponds to catechol derivatives of the following formula 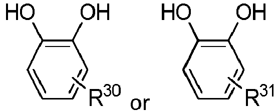 wherein R³⁰ and R³¹ is as described above.

Advantageously, R³⁰ is an electro-withdrawing group (EWG), preferably R³⁰ represent one or several substituent(s), notably 1, 2, 3 or 4 substituent(s), in particular 1 or 4 substituent(s) selected in the group consisting of halogen, such as bromine, and NO₂. According to this embodiment, the method of the present invention will lead to a compound of formula (I) wherein 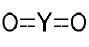 represent a ligand of formula (A).

Advantageously, R³¹ is an electro-donating group (EDG), preferably R³¹ represents one or several H, C₁-C₆ alkyl such as methyl or tert-butyl, or a methoxy group. According to this embodiment, the method of the present invention will lead to a compound of formula (I) wherein 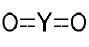 represent a ligand of formula (B).

According to another embodiment, in compound of formula (III), T is O and 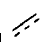 is a double bond. Compound of formula (III) thus corresponds to a pyromeconic acid derivative of the following formula wherein R³² is as described above.

Advantageously, R³² is a C₁-C₆ alkyl, such as a methyl. According to this embodiment, the method of the present invention will lead to a compound of formula (I) wherein 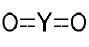 represent a ligand of formula (C).

### Step (ii)

During step (ii), the reaction mixture resulting from step (i) is contacted with an oxidant such as O₂, in particular by opening the reaction medium to air.

Advantageously, the contact of the reaction mixture with O₂ occurs for a time comprised between 1 and 5 h, preferably for 2 h, notably in dark.

### Step (iii)

As described above, X⁻ is preferably selected among PF₆⁻, Cl⁻, Br⁻, BF₄⁻, (C₁-C₆ alkyl)-C(O)O⁻, (C₁-C₆ haloalkyl)-C(O)O⁻, (C₁-C₆ alkyl)- SO₃⁻ and (C₁-C₆ haloalkyl)-SO₃⁻, in particular PF₆⁻, Cl⁻, Br⁻, BF₄⁻, CH₃C(O)O⁻ , CF₃C(O)O⁻ and CF₃SO₃⁻, more preferably X⁻ is PF₆⁻. A⁺ is a counter cation preferably selected among N⁺R'R"R"'R"" (e.g. NH₄⁺, NBu₄⁺), H⁺, Na⁺, K⁺ and Li⁺. The salt A⁺X⁻ is thus preferably selected among the salts, but not limited to, NH₄PF₆, NBu₄PF₆, KCI, KBr, LiCI, LiBr, HBF₄, NaOC(O)CH₃, KOC(O)CH₃, NH₄OCOCH₃. Preferably, the salt used in step (iii) is NH₄PF₆.

Step (iii) is typically achieved when the step (ii) results in "non-neutral" compound of formula (I), i.e. compound wherein 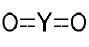 corresponds to form B or C.

Compound of formula (I) wherein 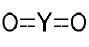 is of formula B or C and m=+1 is then recovered.

The compound obtained can be separated from the reaction medium by methods well known to the person skilled in the art, such as by extraction, evaporation of the solvent or by precipitation or crystallisation (followed by filtration).

The compound can be also purified if necessary by methods well known to the person skilled in the art, such as by recrystallisation, by distillation, by chromatography on a column of silica gel or by high performance liquid chromatography (HPLC).

### Description of the figures

**Figure 1****:** Ruthenium cellular uptake (left) and fractionated subcellular uptake of ruthenium (right). The results were obtained by incubating 5 µM of the target ruthenium compound 1 for 2 h in HeLa cells.
**Figure 2****:** Ruthenium cellular accumulation in MRC5 healthy cells.
**Figure 3****:** Induction of apoptosis / necrosis in HeLa cells upon treatment with the target compound at different time frames. The white bar represents living cells while light grey displays early apoptotic events, dark grey late apoptotic events and black necrotic cells.
**Figure 4****:** Kaplan-Meier analysis of survival. The compounds were administered i.p. on days 1 and 7 after tumour inoculation, n = 7 in each group.
**Figure 5****:** The weight of the solid Ehrlich tumour (in grams) on day 10 of mice injected on days 1 and 7 i.p. with pure vehicle, compound **1** or cisplatin. Values are the means ± SEM (n = 7 in each group). Control - tumour-bearing control treated with mixture of co-solvent and water; Pt - cisplatin 5 mg/kg i.p.; Ru 5-compound **1** 5 mg/kg i.p.; Ru 10 - compound **1** 10 mg/kg i.p.; Ru 15-compound **1** 15 mg/kg i.p. Significantly different from the controls (**P < 0.01).

### Examples

### 1. Synthesis of compounds according to the present invention

### Materials.

All chemicals were either of reagent or analytical grade and used as purchased from commercial sources without additional purification. Ruthenium trichloride hydrate was provided by I²CNS, 4,7-Diphenyl-1,10-phenanthroline, Lithium chloride (anhydrous, 99%), and catechol by Alfa Aesar, tetrabutylammonium hexafluorophosphate by Sigma-Aldrich. All solvents were purchased of analytical, or HPLC grade. When necessary, solvents were degassed by purging with dry, oxygen-free nitrogen for at least 30 min before use.

### Instrumentation and methods.

Amber glass or clear glassware wrapped in tin foil were used when protection from the light was necessary. Schlenk glassware and a vacuum line were employed when reactions sensitive to moisture/oxygen had to be performed under nitrogen atmosphere. Thin layer chromatography (TLC) was performed using silica gel 60 F-254 (Merck) plates with detection of spots being achieved by exposure to UV light. Column chromatography was done using Silica gel 60-200 µm (VWR). Eluent mixtures are expressed as volume to volume (v/v) ratios. ¹H and ¹³C NMR spectra were measured on Bruker Avance III HD 400 MHz or Bruker Avance Neo 500 MHz spectrometers using the signal of the deuterated solvent as an internal standard.⁹ The chemical shifts *δ* are reported in ppm (parts per million) relative to tetramethylsilane (TMS) or signals from the residual protons of deuterated solvents. Coupling constants J are given in Hertz (Hz). The abbreviation for the peaks multiplicity is br (broad). ESI-HRMS experiments were carried out using a LTQ-Orbitrap XL from Thermo Scientific (Thermo Fisher Scientific, Courtaboeuf, France) and operated in positive ionization mode, with a spray voltage at 3.6 kV. Sheath and auxiliary gas were set at a flow rate of 5 and 0 arbitrary units (a.u.), respectively. Applied voltages were 40 and 100 V for the ion transfer capillary and the tube lens, respectively. The ion transfer capillary was held at 275°C. Detection was achieved in the Orbitrap with a resolution set to 100,000 (at m/z 400) and a m/z range between 200-2000 in profile mode. Spectrum was analysed using the acquisition software XCalibur 2.1 (Thermo Fisher Scientific, Courtaboeuf, France). The automatic gain control (AGC) allowed accumulation of up to 2.105 ions for FTMS scans, Maximum injection time was set to 300 ms and 1 µscan was acquired. 5µL was injected using a Thermo Finnigan Surveyor HPLC system (Thermo Fisher Scientific, Courtaboeuf, France) with a continuous infusion of methanol at 100 µL.min-1. Electrospray Ionization-Mass Spectrometry (ESI-MS) experiments were carried out using a LTQ-Orbitrap XL from Thermo Scientific (Thermo Fisher Scientific, Courtaboeuf, France) and operated in positive ionization mode, with a spray voltage at 3.6 kV. No sheath and auxiliary gas were used. Applied voltages were 40 and 100 V for the ion transfer capillary and the tube lens, respectively. The ion transfer capillary was held at 275 °C. Detection was achieved in the Orbitrap with a resolution set to 100,000 (at m/z 400) and an m/z range between 150 and 2000 in profile mode. Spectrum was analyzed using the acquisition software XCalibur 2.1 (Thermo Fisher Scientific, Courtaboeuf, France). The automatic gain control (AGC) allowed for the accumulation of up to 2 x 10⁵ ions for Fourier Transform Mass Spectrometry (FTMS) scans, maximum injection time was set to 300 ms and 1 µs scan was acquired. 10 µL was injected using a Thermo Finnigan Surveyor HPLC system (Thermo Fisher Scientific, Courtaboeuf, France) with a continuous infusion of methanol at 100 µL·min⁻¹. The ATR-FTIR spectra were collected with a dry-air-purged Thermo Scientific Nicolet 6700 FT-IR equipped with a MCT detector. Spectral resolution was 4 cm⁻¹ and spectra were averaged from 256 scans. A horizontal diamond-coated ZnSe crystal with a single reflection and an angle of incidence of 45° was used. A small amount of solid was pressed using a dynanometric stainless steel pressure arm. Elemental analysis was performed at Science Centre, London Metropolitan University using Thermo Fisher (Carlo Erba) Flash 2000 Elemental Analyser, configured for %CHN. IR spectra were recorded with SpectrumTwo FTIR Spectrometer (Perkin-Elmer) equipped with a Specac Golden GateTM ATR (attenuated total reflection) accessory; applied as neat samples; 1/λ in cm-1. Analytical HPLC measurement was performed using the following system: 2 x Agilent G1361 1260 Prep Pump system with Agilent G7115A 1260 DAD WR Detector equipped with an Agilent Pursuit XRs 5C18 (100Å, C18 5 µm 250 x 4.6 mm) Column and an Agilent G1364B 1260-FC fraction collector. The solvents (HPLC grade) were millipore water (0.1% TFA, solvent B) and acetonitrile (0.1% TFA, solvent A). The flow rate was 1 mL/min. Detection was performed at 215nm, 250nm, 350nm, 450nm, 550nm and 650nm with a slit of 4nm. ICP-MS measurements were performed on an Agilent QQQ 8800 Triple quad ICP-MS spectrometer (Agilent Technologies) with a ASX200 autosampler (Agilent Technologies), equipped with standard nickel cones and a "micro-mist" quartz nebulizer fed with 0.3 ml/min analytic flow (as a 2% HNO₃ aqueous solution).

### Abbreviations.

DCM: dichloromethane
DIP: 4,7-diphenyl-1,10-phenanthroline
DMEM: Dulbecco's Modified Eagle Medium
DMF: dimethylformamide
DMSO: dimethyl sulfoxide
ESI: electrospray ionisation
HRMS: High resolution mass spectroscopy
ICP-MS: Inductively Coupled Plasma Mass Spectrometry
IR: Infrared
NMR: Nuclear magnetic resonance
r.t: room temperature

### Synthesis and characterization.

**Ru(DMSO)₂Cl₂** Ru(DM)SO)₂Cl₂ was synthesised following an adapted literature procedure (Brastos, I *et al.,* 2010) Spectroscopic data (¹H NMR) was in agreement with literature.

**Ru(DIP)₂Cl₂.** The complex was synthesised following an adapted literature procedure (Caspar, R. *et al.,* 2006). A mixture of Ru(DMSO)₂Cl₂ (3.0 g, 6.19 mmol), DIP (4,7-diphenyl-1,10-phenanthroline) (4.11 g, 12.38 mmol) and LiCI (2.0 g, 47.18 mmol) dissolved in DMF (100 mL) was refluxed for 24 h. After cooling to room temperature, the solvent was reduced *in vacuo* to 8 mL and 350 mL of acetone were added. The mixture was then stored at -20 °C overnight before filtration with a Buchner funnel and washed with Acetone and Et₂O to afford Ru(DIP)₂Cl₂ as a deep purple solid (3.76 g, 4.49 mmol, 72%). Spectroscopic data (¹H NMR) were in agreement with literature (Caspar, R. *et al.,* 2006).

### Compound 1 ([Ru(DIP)₂(sq)](PF₆))

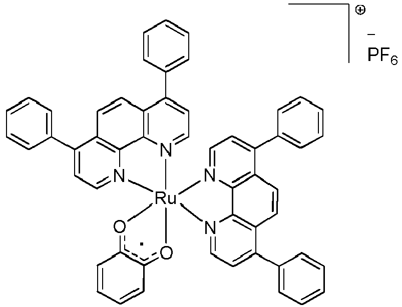

Ru(DIP)₂Cl₂ (0.739 g, 0.88 mmol) and aq. NaOH (0.5 mL, 1 M) were dissolved in 2-propanol (40 mL). The solution was degassed for 15 min and catechol (0.155 g, 1.41 mmol) was added. The mixture was heated to reflux for 24 h under N₂ atmosphere and protected from light. After cooling to *r.t.,* the mixture was stirred opened to air while still protected from light and the solvent was removed under vacuum. The residual solid was dissolved in 2-propanol (7 mL) and H₂O (56 mL) and NH₄PF₆ (0.700 g, 4.3 mmol) were added. The mixture was stored in the fridge (4°C) overnight. The precipitate was filtered with a Buchner funnel and washed with H₂O (3 x 50 mL) and Et₂O (3 x 50 mL). The solid was collected with DCM and dried under vacuum to deliver a crude product as the PF₆ salt (0.70 g), which was chromatographed on silica (DCM/MeCN 20:1 R*f*: 0.3). Evaporation of the solvent under vacuum provided 1 as a deep red solid. Further wash with Et₂O and Heptane were necessary in order to obtain clean product. The solid with the washing solvent (10 mL) was sonicated for 10 min and then centrifuged. This procedure was repeated three times for each solvent. Finally the red solid was collected with DCM and dried under vacuum to afford a clean product (0.17 g, 0.167 mmol, 19%). IR (Golden Gate, cm⁻¹): 3345w, 1710m, 1600w, 1520s, 1455s, 1335s, 1270s, 1125s, 820s, 760m. ¹H NMR (400 MHz, CD₂Cl₂): δ/ppm = 8.79-8.20 (br, 5H, *arom*.), 8.09-7.88 (br, 5H, *arom*.), 7.73-7.42 (br, 14H, *arom*.), 7.26-6.92 (br, 10H, *arom*.), 6.92 - 6.63 (br, 2H, *arom*.). ¹³C NMR (125 MHz, CD₂Cl₂): δ/ppm = 149.84, 144.68, 136.10, 133.56, 130.36, 129.89, 129.53, 128.41, 126.21, 125.36, 121.47, 116.35. For the quaternary carbons, only two were observed in the ¹³C NMR spectrum where five were expected. This could be explained by peak overlap or the signal being too weak to be detected within the acquisition time of the experiment which is common for quaternary carbons. HRMS (ESI+): m/z 874.1887 [M - PF₆]⁺. Elemental Analysis: calcd. for C₅₄H₃₆F₆N₄O₂PRu = C, 63.65; H, 3.56; N, 5.50. Found = C, 63.62; H, 3.52; N, 5.45. HPLC: 0-3 minutes: isocratic 90% B (5% A); 3- 25 minutes: linear gradient from 90% B (5% A) to 0% B (100% A); 25-30 minutes: isocratic 0% B (100% A), 30-35 minutes: linear gradient from 0% B (100% A) to 95% B (5% A), T_{R} = 31.304 min.

### Compound 2 ([Ru(DIP)₂(mal)](PF₆))

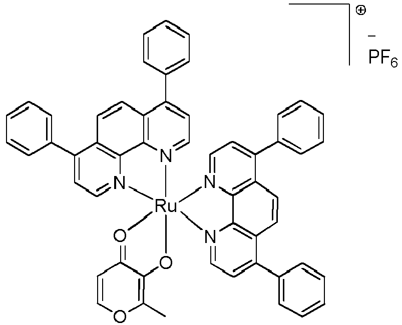

Ru(DIP)₂Cl₂ (0.150 g, 0.18 mmol) and aq. NaOH (0.28 mL, 1 M) were dissolved in ethanol (18 mL). The solution was degassed for 30 min and maltol (3-Hydroxy-2-methyl-4*H*-pyran-4-one) (0.036 g, 0.29 mmol) was added. The mixture was heated to reflux for 3 h under N₂ atmosphere and protected from light. After cooling to *r.t.,* H₂O (200 mL) and NH₄PF₆ (1 g, 6 mmol) were added. The mixture was stored in the fridge (4 °C) overnight. The precipitate was filtered with a Buchner funnel and washed with H₂O (3 x 50 mL), Pentane (3 x 50 mL) and Et₂O (3 x 50 mL). Further wash with Et₂O and Heptane were necessary in order to obtain clean product. The solid with the washing solvent (10 mL) was sonicated for 10 min and then centrifuged. This procedure was repeated three times for each solvent. The solid was collected with DCM and dried under vacuum to deliver a clean product as the PF₆ salt (0.17 g, 0.16 mmol, 90%). IR (Golden Gate, cm⁻¹): 1590w, 1545w, 1490w, 1465w, 1445w, 1415w, 1400w, 1275w, 1205w, 1085w, 1025w, 915w, 830s, 765s, 735m, 700s. ¹H NMR (400 MHz, CD₂Cl₂): δ/ppm = 9.49 (d, *J* = 5.4 Hz, 1H), 9.33 (d, *J* = 5.5 Hz, 1H), 8.23 (dd, *J* = 9.4, 4.6 Hz, 2H), 8.13 (dd, *J* = 9.4, 1.1 Hz, 2H), 8.04 (d, *J* = 5.6 Hz, 2H), 7.98 (dd, *J* = 13.5, 5.4 Hz, 2H), 7.78 - 7.60 (m, 11H), 7.59 - 7.47 (m, 10H), 7.33 (dd, *J* = 5.6, 3.0 Hz, 2H), 6.54 (d, *J* = 5.1 Hz, 1H), 2.37 (s, 3H). ¹³C NMR (125 MHz, CD₂Cl₂): δ/ppm = 185.00, 159.02, 155.49, 154.10, 154.01, 151.75, 151.47, 151.06, 150.64, 150.17, 149.98, 149.92, 147.66, 147.13, 146.01, 145.71, 136.50, 136.47, 136.23, 136.20, 130.15, 129.86, 129.81, 129.47, 129.44, 129.28, 129.21, 129.10, 128.78, 128.61, 128.55, 128.46, 125.96, 125.82, 125.77, 125.77, 125.69, 125.51, 125.25, 125.05, 112.40, 29.84. HRMS (ESI+): m/z 891.19042 [M - PF₆]⁺. Elemental Analysis: calcd. for C₅₄H₃₉F₆N₄O₄PRu = C, 61.54; H, 3.73; N, 5.32. Found = C, 61.53; H, 3.38; N, 5.17.

### Compound 3 [Ru(DIP)₂(4-methylsq)](PF₆)

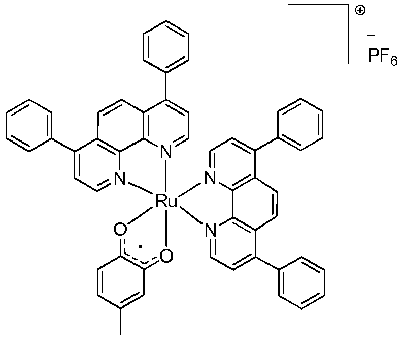

Ru(DIP)₂Cl₂ (0.170 g, 0.20 mmol) and aq. NaOH (0.3 mL, 1 M) were dissolved in 2-propanol (20 mL). The solution was degassed for 15 min and 4-methycatechol (0.05 g, 0.40 mmol) was added. The mixture was heated to reflux for 24 h under N₂ atmosphere and protected from light. After cooling to *r.t.,* the mixture was stirred opened to air for 2h while still protected from light and the solvent was removed under vacuum. The residual solid was dissolved in Ethanol (1 mL) and H₂O (8 mL) and NH₄PF₆ (0.200 g, 1.2 mmol) were added. The mixture was stored in the fridge (4 °C) overnight. The precipitate was filtered with a Buchner funnel and washed with H₂O (3 x 50 mL), Pentane (3 x 50 mL) and Et₂O (3 x 50 mL). The solid was collected with DCM and dried under vacuum to deliver a crude product as the PF₆ salt (0.07 g), which was chromatographed on silica (DCM/MeCN 20:1 R*f* : 0.3). Evaporation of the solvent under vacuum provided [Ru(DIP)₂(4-methylsq)](PF₆) as a deep red solid. Further wash with Et₂O and Heptane were necessary in order to obtain clean product. The solid with the washing solvent (10 mL) was sonicated for 10 min and then centrifuged. This procedure was repeated three times for each solvent. Finally the red solid was collected with DCM and dried under vacuum to afford a clean product (0.06 g, 0.06 mmol, 29%). IR (Golden Gate, cm⁻¹): 3060w, 1620w, 1590, 1560, 1510w, 1420m, 1240m, 1120w, 1090w, 1030w, 912w, 827s, 762s, 735m, 698s. ¹H NMR (500 MHz, CD₂Cl₂) δ 8.63-8.07 (br, 6H, *arom*.), 8.03 - 7.81 (br, 7H, *arom*.), 7.72 - 7.36 (br, 16H, *arom*.), 7.36 - 7.03 (m, 9H, *arom*.). ¹³C NMR (125 MHz,): δ/ppm = 149.08, 147.51, 143.55, 142.50, 140.12, 140.01, 136.57, 136.18, 132.83, 132.35, 130.28, 130.19, 129.97, 129.60, 129.49, 128.89, 128.71, 128.37, 126.41, 124.88, 123.69. MS (ESI+): m/z 888.7 [M - PF₆]⁺. Elemental Analysis: calcd. for C₅₅H₃₈F₆N₄O₂PRu = C, 63.95; H, 3.71; N, 5.42. Found = C, 63.84; H, 3.62; N, 5.29. HPLC: 0-3 minutes: isocratic 65% B (35% A); 3- 17 minutes: linear gradient from 65% B (35% A) to 0% B (100% A); 17-23 minutes: isocratic 0% B (100% A), T_{R} = 13.804 min.

### Compound 4 ([Ru(DIP)₂(3-methoxysq)](PF₆))

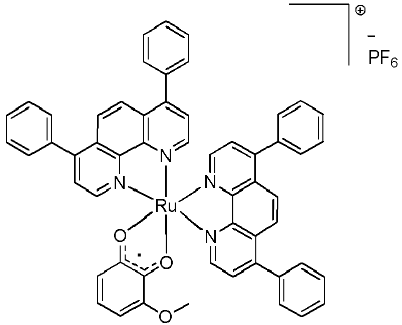

Ru(DIP)₂Cl₂ (0.250 g, 0.29 mmol) and aq. NaOH (0.45 mL, 1 M) were dissolved in 2-propanol (5 mL). The solution was degassed for 15 min and 3-methoxycatechol (0.07 g, 0.5 mmol) was added. The mixture was heated to reflux for 24 h under N₂ atmosphere and protected from light. After cooling to *r.t.,* the mixture was stirred opened to air for 2h while still protected from light and the solvent was removed under vacuum. The residual solid was dissolved in 2-propnol (2.5 mL) and H₂O (20 mL) and NH₄PF₆ (0.250 g, 1.5 mmol) were added. The mixture was stored in the fridge (4°C) overnight. The precipitate was filtered with a Buchner funnel and washed with H₂O (3 x 50 mL), Pentane (3 x 50 mL) and Et₂O (3 x 50 mL). The solid was collected with DCM and dried under vacuum to deliver a crude product as the PF₆ salt (0.07 g), which was chromatographed on silica (DCM/MeCN 20:1 R*f* : 0.3). Evaporation of the solvent under vacuum provided [Ru(DIP)₂(3-methoxysq)](PF₆) as a deep red solid. Further wash with Et₂O and Heptane were necessary in order to obtain clean product. The solid with the washing solvent (10 mL) was sonicated for 10 min and then centrifuged. This procedure was repeated three times for each solvent. Finally the red solid was collected with DCM and dried under vacuum to afford a clean product (0.072 g, 0.06 mmol, 23%). IR (Golden Gate, cm⁻¹):3060w, 1620w, 1590w, 1540w, 1460m, 1400m, 1250m, 1160m, 1100m, 1030w, 827s, 764s, 735m, 700s. ¹H NMR (500 MHz, CD₂Cl₂): δ/ppm = 8.91 - 8.50 (br, 1H, *arom*.), 8.43 - 8.08 (br, 3H, *arom*.), 8.07 - 7.79 (br, 7H, *arom*.), 7.75 - 7.46 (br, 15H, *arom*.), 7.46 - 7.28 (br, 2H, *arom*.), 7.28 - 6.93 (br, 10H, *arom*.). ¹³C NMR (125 MHz, CD₂Cl₂): δ/ppm = 149.62, 146.57, 143.72, 140.55, 137.05, 136.03, 133.07, 132.47, 131.27, 130.31, 130.07, 130.00, 129.59, 129.56, 129.33, 128.97, 128.80, 128.57, 125.67, 125.46, 123.55. MS (ESI+): m/z 904.8 [M - PF₆]⁺. Elemental Analysis: calcd. for C₅₅H₄₀F₆N₄O₄PRu = C, 62.76; H, 3.64; N, 5.53. Found = C, 61.67; H, 3.63; N, 5.09. HPLC: 0-3 minutes: isocratic 65% B (35% A); 3- 17 minutes: linear gradient from 65% B (35% A) to 0% B (100% A); 17-23 minutes: isocratic 0% B (100% A), T_{R} = 11.887 min.

### Compound 5 ([Ru(DIP)₂(tetrabromocat)])

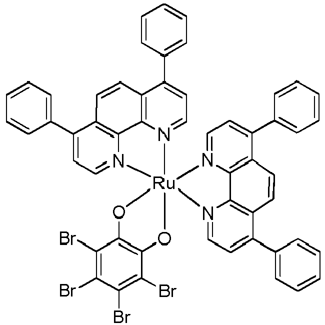

Ru(DIP)₂Cl₂ (0.270 g, 0.32 mmol) and aq. NaOH (0.50 mL, 1 M) were dissolved in 2-propanol (27 mL). The solution was degassed for 15 min and tetrabromocatechol (0.220 g, 0.5 mmol) was added. The mixture was heated to reflux for 24 h under N₂ atmosphere and protected from light. After cooling to *r.t.,* the mixture was stirred opened to air for 2h while still protected from light and the solvent was removed under vacuum. The residual solid was dissolved in 2-propnol (2.5 mL) and H₂O (20 mL) and NH₄PF₆ (0.250 g, 1.5 mmol) were added. The mixture was stored in the fridge (4°C) overnight. The precipitate was filtered with a Buchner funnel and washed with H₂O (3 x 50 mL), Pentane (3 x 50 mL) and Et₂O (3 x 50 mL). The solid was collected with DCM and dried under vacuum to deliver a crude product (0.320 g), which was chromatographed on silica (DCM/Et₂O 98:2 R*f* : 0.8). Evaporation of the solvent under vacuum provided Ru(DIP)₂(tetrabromocat) as a blue solid. Further wash with Et₂O and Heptane were necessary in order to obtain clean product. The solid with the washing solvent (10 mL) was sonicated for 10 min and then centrifuged. This procedure was repeated three times for each solvent. Finally the blue solid was collected with Ethanol and dried under vacuum to afford a clean product (0.209 g, 0.176 mmol, 54%). IR (Golden Gate, cm⁻¹): 3060w, 1600w, 1430s, 1260m, 1080m, 1030w, 914m, 847m, 760m, 731m, 700s. ¹H NMR (400 MHz, CD₂Cl₂): δ/ppm = 8.84 - 8.44 (br, 6H, *arom*.), 8.16- 8.01 (br, 4H, *arom*.), 7.76- 7.62 (br, 8H, *arom*.), 7.62 - 7.41 (br, 11H, *arom*.), 7.41- 7.28 (br, 3H, *arom*.). ¹³C NMR (100 MHz, CD₂Cl₂): δ/ppm = 141.93, 139.70, 131.49, 130.20, 129.81, 129.45, 126.33, 125.79, 125.33. MS (ESI+): m/z 1090.4 [M]⁺. Elemental Analysis: calcd. for C₅₄H₃₂Br₄N₄O₂Ru = C, 54.52; H, 2.71; N, 4.71. Found = C, 54.56; H, 2.37; N, 4.89. HPLC: 0-3 minutes: isocratic 85% B (35% A); 3- 7 minutes: linear gradient from 85% B (35% A) to 0% B (100% A); 7-9 minutes: isocratic 0% B (100% A), HPLC: T_{R} = 8.623 min.

### Compound 6 [Ru(DIP)₂(3-methylsq)](PF₆)

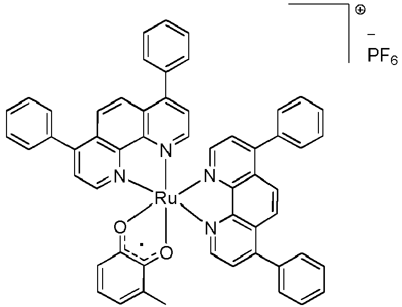

Ru(DIP)₂Cl₂ (0.170 g, 0.20 mmol) and aq. NaOH (0.3 mL, 1 M) were dissolved in 2-propanol (20 mL). The solution was degassed for 15 min and 3-methycatechol (0.05 g, 0.40 mmol) was added. The mixture was heated to reflux for 24 h under N₂ atmosphere and protected from light. After cooling to *r.t.,* the mixture was stirred opened to air for 2h while still protected from light and the solvent was removed under vacuum. The residual solid was dissolved in Ethanol (1 mL) and H₂O (8 mL) and NH₄PF₆ (0.200 g, 1.2 mmol) were added. The mixture was stored in the fridge (4 °C) overnight. The precipitate was filtered with a Buchner funnel and washed with H₂O (3 x 50 mL), Pentane (3 x 50 mL) and Et₂O (3 x 50 mL). The solid was collected with DCM and dried under vacuum to deliver a crude product as the PF₆ salt (0.06 g), which was chromatographed on silica (DCM/MeCN 20:1 R*f* : 0.3). Evaporation of the solvent under vacuum provided [Ru(DIP)₂(4-methylsq)](PF₆) as a deep red solid. Further wash with Et₂O and Heptane were necessary in order to obtain clean product. The solid with the washing solvent (10 mL) was sonicated for 10 min and then centrifuged. This procedure was repeated three times for each solvent. Finally the red solid was collected with DCM and dried under vacuum to afford a clean product (0.05 g, 0.05 mmol, 24%). IR (Golden Gate, cm⁻¹): 3060w, 1600w, 1540m, 1390m, 1250m, 1150m, 1100w, 1030w, 827s, 764s, 735s, 700s. ¹H NMR (400 MHz, CD₂Cl₂) δ 8.79 - 8.11 (br, 6H, *arom*.), 8.08 - 7.82 (br, 6H, *arom*.), 7.60 (br, 15H, *arom*.), 7.40 - 6.81 (br, 11H, *arom*.). ¹³C NMR (100 MHz,): δ/ppm = 148.62, 147.00, 142.78, 142.70, 142.59, 136.94, 136.22, 132.88, 130.54, 130.25, 130.13, 129.79, 129.61, 129.56, 128.64, 126.87, 126.52, 124.93, 124.47, 121.72. MS (ESI+): m/z 888.7 [M - PF₆]⁺. Elemental Analysis: calcd. for C₅₅H₄₀F₆N₄O₃PRu = C, 62.86; H, 3.84; N, 5.33. Found = C, 62.95; H, 3.69; N, 5.20. HPLC: 0-3 minutes: isocratic 65% B (35% A); 3- 17 minutes: linear gradient from 65% B (35% A) to 0% B (100% A); 17-23 minutes: isocratic 0% B (100% A), T_{R} = 13.804 min.

### Compound 7 [Ru(DIP)₂(4-tert-buthylsq)](PF₆).

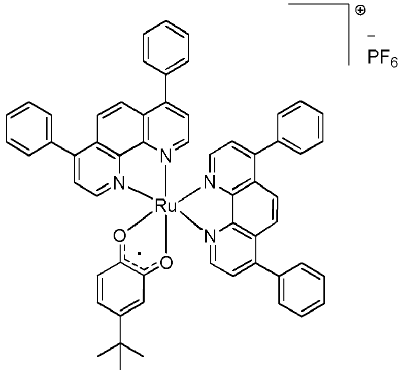

Ru(DIP)₂Cl₂ (0.250 g, 0.29 mmol) and aq. NaOH (0.45 mL, 1 M) were dissolved in 2-propanol (5 mL). The solution was degassed for 15 min and 4-tert-buthylcatechol (0.08 g, 0.5 mmol) was added. The mixture was heated to reflux for 24 h under N₂ atmosphere and protected from light. After cooling to *r.t.,* the mixture was stirred opened to air for 2h while still protected from light and the solvent was removed under vacuum. The residual solid was dissolved in 2-propnol (2.5 mL) and H₂O (20 mL) and NH₄PF₆ (0.250 g, 1.5 mmol) were added. The mixture was stored in the fridge (4°C) overnight. The precipitate was filtered with a Buchner funnel and washed with H₂O (3 x 50 mL), Pentane (3 x 50 mL) and Et₂O (3 x 50 mL). The solid was collected with DCM and dried under vacuum to deliver a crude product as the PF₆ salt (0.08 g), which was chromatographed on silica (DCM/MeCN 20:1 R*f* : 0.3). Evaporation of the solvent under vacuum provided [Ru(DIP)₂(3-methylysq)](PF₆) as a deep red solid. Further wash with Et₂O and Heptane were necessary in order to obtain clean product. The solid with the washing solvent (10 mL) was sonicated for 10 min and then centrifuged. This procedure was repeated three times for each solvent. Finally the red solid was added dropwise to cold stirring Et₂O (200 mL) filtrated, collected with DCM and dried under vacuum to afford a clean product (0.052 g, 0.05 mmol, 16%).IR (Golden Gate, cm⁻¹): 3060w, 2960w, 1620w, 1580w, 1510m, 1450m, 1420m, 1220m, 1090w, 1030w, 827s, 764s,735s, 700s. ¹H NMR (400 MHz, CD₂Cl₂): δ/ppm = 8.43 - 8.12 (br, 6H, *arom*.), 8.09 - 7.81 (br, 9H, *arom*.), 7.70 - 7.46 (br, 16H, *arom*.), 7.46 - 7.24 (br, 4H, *arom*.), 7.24 - 7.04 (br, 8H, *arom*.). ¹³C NMR (125 MHz, CD₂Cl₂): δ/ppm = 147.51, 147.31, 143.70, 136.70, 136.64, 132.77, 132.54, 130.21, 129.63, 129.45, 128.84, 128.75, 127.49, 126.60, 124.85, 124.45. MS (ESI+): m/z 930.8 [M - PF₆]⁺. Elemental Analysis: calcd. for C₅₈H₄₄F₆N₄O₂PRu = C, 64.80; H, 4.13; N, 5.21. Found = C, 64.72; H, 4.13; N, 5.14.

### Compound 8 (Ru(DIP)₂(4-nitrocat)).

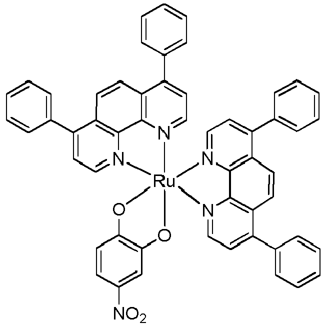

Ru(DIP)₂Cl₂ (0.270 g, 0.32 mmol) and aq. NaOH (0.50 mL, 1 M) were dissolved in 2-propanol (27 mL). The solution was degassed for 15 min and 4-nitrocatechol (0.073 g, 0.5 mmol) was added. The mixture was heated to reflux for 24 h under N₂ atmosphere and protected from light. After cooling to *r.t.,* the mixture was stirred opened to air for 2h while still protected from light and the solvent was removed under vacuum. The residual solid was dissolved in 2-propnol (2.5 mL) and H₂O (20 mL) and NH₄PF₆ (0.250 g, 1.5 mmol) were added. The mixture was stored in the fridge (4 °C) overnight. The precipitate was filtered with a Buchner funnel and washed with H₂O (3 x 50 mL), Pentane (3 x 50 mL) and Et₂O (3 x 50 mL). The solid was collected with DCM and dried under vacuum to deliver a crude product (0.120 g), which was chromatographed on silica (DCM/MeOH 96:4 R*f* : 0.4). Evaporation of the solvent under vacuum provided Ru(DIP)₂(4-nitrocat) as a deep blue solid. Further wash with Et₂O and Heptane were necessary in order to obtain clean product. The solid with the washing solvent (10 mL) was sonicated for 10 min and then centrifuged. This procedure was repeated three times for each solvent. Finally the blue solid was added dropwise to cold stirring Et₂O (200 mL) filtrated, collected with DCM and dried under vacuum to afford a clean product (0.209 g, 0.176 mmol, 54%). IR (Golden Gate, cm⁻¹): 3060w, 1550w, 1490m, 1410w, 1240s, 1120m, 1070s, 1030w, 949w, 910w, 845s, 762s, 733s, 698s. ¹H NMR (400 MHz, CD₂Cl₂): δ/ppm = 9.65 - 9.21 (br, 3H, *arom*.), 8.24 - 7.85 (br, 8H, *arom*.), 7.81 - 7.40 (br, 17H, *arom*.), 7.41 - 7.18 (br, 2H, *arom*.), 6.87 - 6.59 (br, 1H, *arom*.), 6.60 - 6.27 (br, 2H, *arom*.), 6.11 - 5.84 (br, 2H, *arom*.). ¹³C NMR (125 MHz, CD₂Cl₂): δ/ppm = 153.27, 152.64, 152.53, 150.58, 146.76, 146.65, 144.72, 144.63, 137.40, 137.17, 130.79, 130.69, 130.19, 129.61, 129.50, 129.44, 128.86, 128.38, 126.17, 125.87, 125.49, 125.15, 124.99, 124.87. MS (ESI+): m/z 919.4 [M]⁺. Elemental Analysis: calcd. for C₅₄H₃₉N₅O₆Ru = C, 67.92; H, 4.12; N, 7.33. Found = C, 68.04; H, 4.11; N, 7.28. HPLC: 0-3 minutes: isocratic 65% B (35% A); 3- 17 minutes: linear gradient from 65% B (35% A) to 0% B (100% A); 17-23 minutes: isocratic 0% B (100% A), T_{R} = 15.907 min.

### 2. Cytotoxicity Studies

The cytotoxicity of compounds according to the present invention toward the following cells: HeLa (human cervical), A2780 (human ovarian carcinoma), A2780 cis (human cisplatin resistant ovarian carcinoma), A2780 ADR (human doxorubicin resistant ovarian carcinoma), U87 (human brain glioblastoma), CT-26 (mouse colon adenocarcinoma), CT-26 LUC (mouse colon adenocarcinoma stably expressing luciferase) and RPE-1 (normal retina pigmented epithelial) cell lines was therefore investigated using a fluorometric cell viability assay (Frei et *al.,* 2014). Cytotoxicity of cisplatin and doxorubicin was also determined in the same cell lines as positive controls.

### Cell culture

HeLa and CT-26 cell lines were cultured in DMEM media (Gibco) supplemented with 10% of fetal calf serum (Gibco). CT-26 LUC cell line was cultured in DMEM media (Gibco) supplemented with 10% of fetal calf serum (Gibco) and 1% Genticin. RPE-1 cell line was cultured in DMEM/F-12 media (Gibco) supplemented with 10% of fetal calf serum. A2780, A2780 cis, A2780 ADR cell lines were cultured in RPMI 1640 media (Gibco) supplemented with 10% of fetal calf serum (Gibco). The resistance of A2780 cis was maintained by cisplatin treatment (1µM) for one week every month. The cells were used in the assays after one week from the end of the treatment in order to avoid interfered results. The resistance of A2780 ADR was maintained by doxorubicin treatment (0.1 µM) once a week. Cells were used in the assays after three days post doxorubicin treatment in order to avoid interfered results. U87 cell line was cultured in MEM media (Gibco) supplemented with 10% of fetal calf serum (Gibco) and 1% of non-essential amino acid mixture (Gibco). Cell lines were complemented with 100 U/ml penicillin-streptomycin mixture (Gibco) and maintained in humidified atmosphere at 37°C and 5% of CO₂.

### Cytotoxicity assay

Cytotoxicity of the tested Ru compounds according to the present invention was assessed by a fluorometric cell viability assay using Resazurin (ACROS Organics). Briefly, cells were seeded in triplicates in 96-well plates at a density of 4 x 10³ cells/well in 100 µL in medium. After 24 h, cells were treated with increasing concentrations of the compounds. Compounds dilutions were prepared as follows: 2.5 mM stock in DMSO was further diluted to 100 µM with media and then filtrated. After 48 h incubation, medium was removed, and 100 µl of complete medium containing resazurin (0.2 mg/ml final concentration) was added. After 4 h of incubation at 37 °C, the fluorescence signal of resorufin product was read (ex: 540 nm em: 590 nm) in a SpectraMax M5 microplate Reader. IC₅₀ values were then calculated using GraphPad Prism software.

### Results and discussion

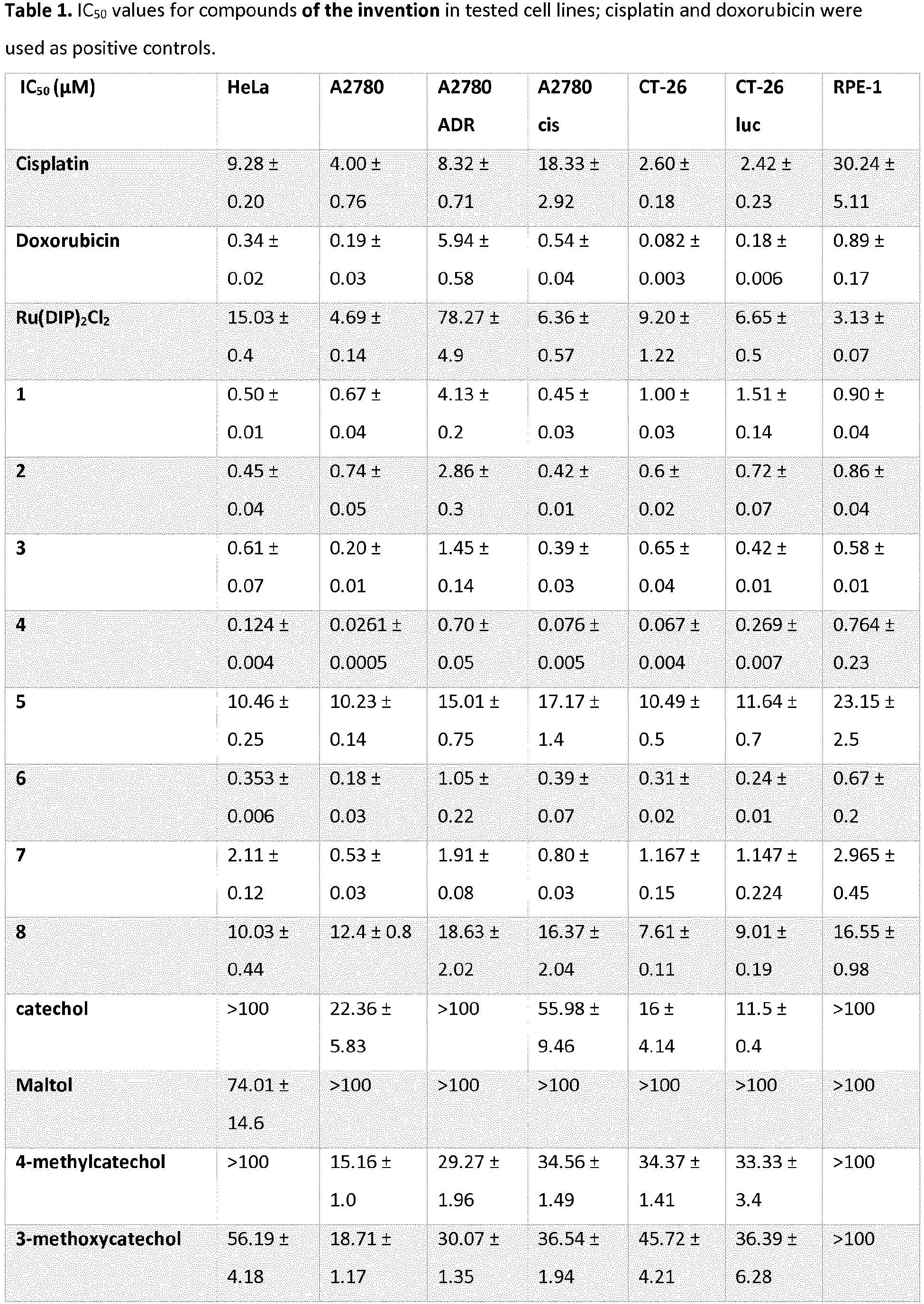

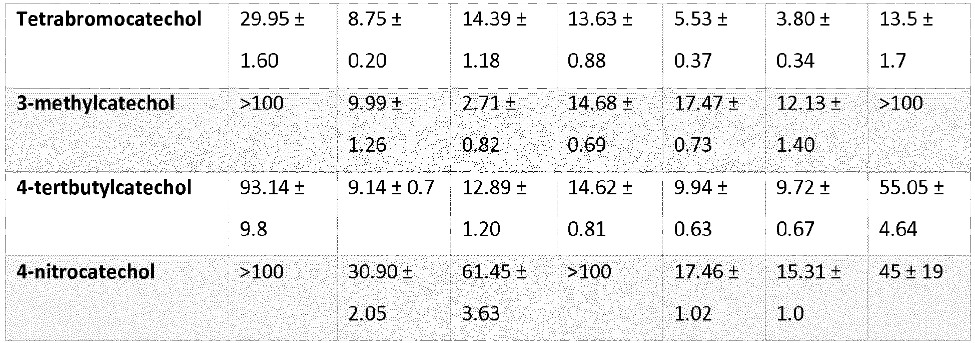

It is striking that the 1 compound has IC₅₀ values (the half maximal inhibitory concentration) in the nanomolar range for most of the cell lines investigated in this study, even for cisplatin resistant cell line with a value of almost 100 times lower than cisplatin itself. On the other hand, cytotoxicity of doxorubicin and compound **1** against the doxorubicin resistant cell line appear to be in the same order of magnitude. The analogue carrying the maltol group (**2**) shows comparable activity against most of the cell lines tested. Comparing **1** with the derivatives carrying the EDG (**3, 4, 6, 7**), it is clear how the electron density on the organic moiety plays a role. In general compounds **3, 4, 6, 7** present higher cytotoxicity in most of the cell lines tested. Of particular interest is compound **4** with an IC₅₀ in the low nanomolar range against the cisplatin resistant cell line which makes it 10 times more active than doxorubicin and around 200 times more active than cisplatin. **4,** unlike **1,** presents an IC₅₀ of 0.7 µM against the doxorubicin resistant cell line which is 10 times lower than **1** and cisplatin. Overall, compounds **1, 3, 4, 6,** 7 display a cytotoxicity which is comparable to doxorubicin and which is much higher than cisplatin, with **4** showing the best potential due to its exceptionally low cytotoxicity and its great activity towards resistant cell lines.

compounds carrying the EWG groups (**5, 8**) show much less cytotoxicity than their charged analogues mentioned above with IC₅₀ in the micro molar range for all the cell lines tested.

Catechol, catechol derivatives, maltol and the precursor complex Ru(DIP)₂Cl₂ were tested against the same cell lines as a control. From these results, it is possible to conclude that the great activity shown by compounds **1, 2, 3, 4, 6** and **7** is the consequence of the coordination of the electron rich organic ligands to the main Ru(II) polypyridyl core.

### 3. Cellular uptake

To have more insights about cellular uptake efficiency and cellular accumulation of compound **1,** ICP-MS experiments on HeLa and MRC5 cells were performed.

### Sample Preparation for in vitro ICP-MS.t

HeLa and MRC5 cells were seeded a week before treatment at a concentration of 1x10⁶ cells/ml in 15 cm² cell culture Petri dish, let grow until 80% of confluence and incubated with the target compound (previously dissolved in ethanol as vehicle, v/v < 0.1%) at a concentration of 5.0 µM for 6 hours. Working concentration and incubation time have been chosen in order to avoid extended cell mass lost due to the high cytotoxicity of the compounds but considering a Ru final amount that could afford a significant determination of the metal content. All the treatments were set up at the same concentration for comparative purposes. The medium was then removed, the cells washed with PBS and trypsinized. After re-suspension in PBS, the pellet was washed with ice cold PBS and collected per centrifugation (5910R, Eppendorf) at 500 g for 5 min at 4 °C. The organelles were then isolated via differential centrifugation. To separate lysosomes, a Lysosome Isolation Kit (Cat. Nr: LYSISO1, Sigma Aldrich) as well as the manufacturer procedure with minor modification was used. Briefly, the collected pellets were redissolved in 2.0 ml of extraction buffer added with proteases cocktail (delivered with the kit) and incubated for 15 min on ice. The samples were then homogenized with a pre-chilled dounce homogenizer (7 ml, tight pestle A, 22 strokes) and centrifuged at 1000 g for 10 min at 4 °C. After homogenization, the pellet obtained was redissolved in 2 ml of a sucrose solution (0.25 M sucrose, 10 mM MgCl₂) and layered with 2 ml of a second hypertonic sucrose solution (0.55 M sucrose, 0.5 mM MgCl₂). The suspension was centrifuged at 1450 g and 4 °C for 5 min. The pellet was resuspended in 3 ml of the second sucrose solution and centrifuged at 1450 g and 4 °C for 5 min to obtain the nuclear extract. These steps of the isolation procedure were monitored under phase contrast microscope on Menzel-Gläser coverslips (Olympus IX81 microscope).

The supernatant was transferred in a fresh tube and centrifuged at 14000 g for 30 min at 4 °C. The pellet collected, dissolved in 1 mL of a 19% Optiprep Density Gradient Medium (present in the kit), added with a 250 mM solution of CaCl₂ (final concentration 8 mM) and centrifuged at 5000 g for 15 minutes at 4 °C to give the isolated lysosomal suspension. The intact lysosomal content was proofed with Neutral-Red Reagent (delivered in the kit) with dual-wavelength absorbance mode at 460 and 510 nm over a kinetic of 3 minutes in a Spectramax M5 microplate reader (Molecular Devices). Mitochondria fractions were isolated via further differential centrifugation from the pellet non-containing lysosomes using a mitochondria extraction buffer (Cat. Nr.: E2778, Sigma Aldrich) following the manufacturer instructions and a recent public procedure. The samples were re-dissolved in 1.5 mL of extraction buffer and centrifuged at 11000 g for 10 minutes at 4 °C to obtained final pellets represented pure mitochondrial fractions.

All the fractions (mitochondria, lysosomes and nucleus) were isolated from the same cellular sample for direct comparative purposes. The supernatant phases discarded during the isolation of nuclei, lysosomes and mitochondria procedures were collected and formed the "residual" fraction. An aliquot of crude lysate after homogenization, nuclear, mitochondrial (pellet lysed via freeze and thaw cycles followed by 20 minutes incubation in ultrasonic bath), lysosomal and residual fraction was each used for protein quantification using the Bradford method. The isolated samples were then lyophilized on an Alpha 2-4 LD plus (CHRIST). The resulting samples underwent chemical digestion with 10 mL of a 2% nitrohydrochloric acid solution for 24 h. The resulting suspensions were filtered on 0.20 µm non-pyrogenic sterile Filtropur filters (Sarstedt) and the obtained samples were injected in ICP-MS.

### In vitro ICP-MS Studies.

Ruthenium was measured against a Platinum single element standard (Merck 1703410100) and verified by a control (Agilent5188-6524 PA Tuning 2). Ruthenium content of the samples was determined by means of a 7-step serial dilution in the range between 0 and 300 ppb in Ru (R>0.99) with a background equivalent concentration of BEC: 6.3 ppt and a detection limit of DL: 17 ppt. Spiking the samples with untreated negative controls (to account for eventual carbon content from the biological samples) resulted in equivalent values within error ranges. A solution of Indium (500 ppb) and Tungsten (500 ppb) was used as internal standard. The results are expressed as ng Pt / mg protein (correction due to the different mass of the observed cellular compartments), as mean ± standard deviation error of different independent experiments.

### Results and discussion

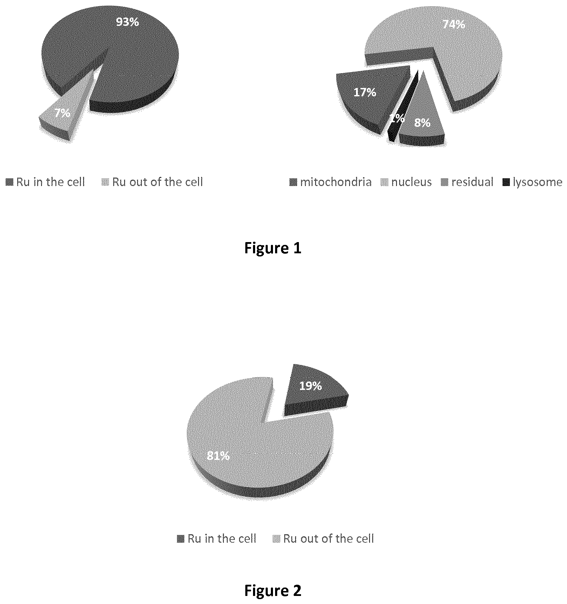

To have more insights about the possible mode of action of compound **1** and its different activity between healthy and cancer cells the, ICP-MS experiments on HeLa and MRC5 cells were performed. Near complete internalization was found to occur in HeLa after 2 h with preferential accumulation inside the nucleus (see Figure 1, right). The uptake is consistent with similar studies on ruthenium polypyridyl complexes which suggests a passive diffusion mechanism due to the lipophilicity inferred by the large aromatic ligand scaffold (Puckett, C.A. et al., 2008). Furthermore, polypyridyl containing ruthenium complexes are capable of intercalating with the DNA (Puckett, C.A., 2009). Preferential accumulation inside the nucleus suggests that the mode of action is related to the damage of the DNA and/or prevention of replication as well as transcription. The same ICP-MS experiment was repeated in MRC-5 (normal lung fibroblasts) cell line (Figure 2). In this case, less than 25% of the tested compound was taken up by healthy cells. This interesting behaviour strongly suggests a preference for compound **1** to accumulate into cancer cells.

### 4. Cell Death Mechanism

To further understand the mode of action of compound **1,** it was important to evaluate the type of cell death that it was causing. For this experiment, Annexin V and PI staining method were utilised on HeLa cells.¹⁵ Staurosporin, a known inducer of apoptosis, was used as a positive control.

### Material and methods

### Annexin V / PI assay.

The apoptosis and necrosis induction in HeLa cells treated with 3 was evaluated via by means of the well-established AnnexinV / PI assay using flow cytometry and according to the manufacturer instructions with just minor changes (Sigma Aldrich, Cat. N°:APOAF). Briefly, the cells were seeded in a 100x15 mm Petri dish at a concentration of 2 x 10⁵ and cultured at 37°C / 6% CO₂ for 24 h. The medium was then removed and replaced with fresh medium containing 10 µM solution of medium containing compound **1** and further incubated for 30 min, 4 h or 24 h. The cells were then trypsinized and pelleted, washed twice with ice cold PBS, centrifuged at 600g for 5 minutes, resuspended in 500 µL binding buffer (provided with the kit) and transferred FACS culture tube. 7.5 µL of Annexin V FITC complex solution (provided with the kit) and 10 µL of propidium iodide (PI) solution (provided with the kit) were added. The samples were incubated for 15 minutes at room temperature (25°C) in the dark, 1000 µL of binding buffer were added and the probes analyzed with a CynAn ADP9 flow cytometer with the FITC (for Annexin V-FITC, excitation = 488 nm, emission = 515-545 nm) and PE-Texas Red channels (for PI, excitation = 488 nm, emission = 564-606 nm) and. The data were analyzed with Summit v4.3 software. Positive controls of cells treated with 0.1 µM or 1.0 µM of staurosporine for 4 h or 24 h recovery respectively were performed.

### Results and discussion

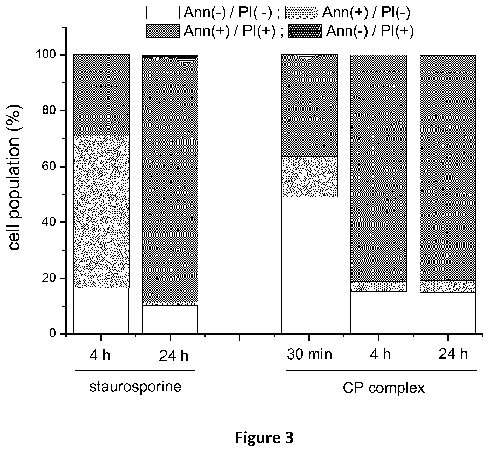

As shown in Figure 3, even 30 min after treatment with compound **1** induces apoptosis in treated cells. Further incubation of the cells with the Ru compound (4 h or 24 h) still points out for the apoptosis as a main type of the cell death.

### 5. Metabolic Studies

The preference of compound **1** to accumulate in the nucleus and mitochondria in HeLa cells inspired further studies on metabolic pathways that could be affected by the compound. For this purpose, Seahorse XF Analyzer was used. Firstly, the effect of compound **1** on the mitochondrial metabolism (oxidative phosphorylation) in CT-26 cell line was investigated. The effect on the other metabolic pathways, i.e. glycolysis and the possible metabolic modulation of the three primary fuel pathways, i.e. glucose, glutamine and fatty acid were then examined.

### Material and methods

### Mitostress Test.

CT-26 cells were seeded in Seahorse XFe96 well plates at a density of 30,000 cells/ well in 80 µL. After 24 h, the media was replaced with fresh media and cisplatin (10 µM), catechol (10 µM), Ph₂Phen (1 µM), complex Ru(DIP)₂Cl₂ (10 µM) or compound **1** (1 µM) were added. After 24 h of incubation, the regular media was removed and the cells were washed thrice using bicarbonate and serum free DMEM, supplemented with glucose, 1.8 mg/ mL; 1% glutamine and 1% sodium pyruvate and incubated in a non-CO₂ incubator at 37 °C for 1 h. Mitostress assay was run using Oligomycin, 1 µM, FCCP 1 µM and mixture of Antimycin-A/ Rotenone 1 µM each in ports A, B and C respectively using Seahorse XFe96 Extracellular Flux Analyzer.

### Seahorse Glycolytic Stress Test.

CT-26 cells were seeded in Seahorse XFe96 well plates at a density of 30,000 cells/ well in 80 µL. After 24 h, the media was replaced with fresh media and cisplatin (10 µM), catechol (10 µM), Ph₂Phen (1 µM), complex Ru(DIP)₂Cl₂ (10 µM) or compound **1** (1 µM) were added. After 24 h of incubation, the regular media was removed and the cells were washed thrice using bicarbonate, glucose and serum free DMEM, supplemented 1% glutamine and 1% sodium pyruvate and incubated in a non-CO₂ incubator at 37 °C for an hour. Glycolytic stress test was run using glucose, 10 mM, Oligomycin, 1 µM and 2-Deoxyglucose, 50 mM in ports A, B and C respectively using Seahorse XFe96 Extracellular Flux Analyzer.

### Fuel Flex Assay.

CT-26 cells were seeded in Seahorse XFe96 well plates at a density of 30,000 cells/ well in 80 µL. After 24 h, the media was replaced with fresh media and cisplatin (10 µM), catechol (10 µM), Ph₂Phen (1 µM), complex Ru(DIP)₂Cl₂ (10 µM) or compound **1** (1 µM) were added. After 24 h of incubation, the regular media was removed and the cells were washed thrice using bicarbonate, and serum free DMEM, supplemented with 1.8 mg/mL glucose, 1% glutamine and 1% sodium pyruvate and incubated in a non-CO₂ incubator at 37 °C for an hour. Fuel flex assay for the different fuel pathways viz. glucose, glutamine and fatty acid was studied by measuring the basal oxygen consumption rates and that after addition of the inhibitor of the target pathway in port A and a mixture of the inhibitors of the other two pathways in port B. This gave a measure of the dependency of the cells on a fuel pathway. To study the capacity of a certain fuel pathway, the sequence of addition of the inhibitors was reversed. In port A was added the mixture of inhibitors for the other pathways and in port B was added the inhibitor for the target pathway. UK-5099 (pyruvate dehydrogenase inhibitor, 20 µM) was used as an inhibitor for the glucose pathway. BPTES (selective inhibitor of Glutaminase GLS1, 30 µM) was used as an inhibitor for the glutamine pathway. Etomoxir (O-carnitine palmitoyltransferase-1 (CPT-1) inhibitor, 40 µM) was used as an inhibitor for the fatty acid pathway.

### Results and discussion

Mitochondrial respiration was found to be severely impaired in cells treated with compound **1** as opposed to the precursor of complex **1.** This was clear from the low basal respiration, compared to the untreated cells. ATP production was also inhibited by compound **1.** The mitochondrial membrane lost the capacity to restore the proton balance when treated with an uncoupling agent FCCP. All these effects point to the disrupted mitochondrial respiration in mouse colon carcinoma caused by the complex. In contrast, the glycolysis of the cells, which is a cytosolic process, is not affected by compound **1.** Moreover, no direct effect on the 3-primary fuel pathways was shown by compound **1.**

### 6. In vivo studies

Next, *in vivo* studies were performed where both the effect on tumour growth and that on the survival of tumour-bearing mice of an immunocompetent strain were evaluated. The doses were selected according to the dose-finding study, which had revealed a maximum tolerated dose (MTD) of 15 mg/kg of body weight.

### Material and methods

### Animals and Tumour Model.

Due to the poor solubility of compound **1** in water, dimethyl sulfoxide (DMSO), 1.81 ml / kg of body weight, had to be added to water for injections, for which reason the i.p. route of administration was chosen rather than i.v. Female outbred mice (NMRI) were used for this study, they were obtained from Masaryk University (Brno, Czech Republic). Animal care was conformed to EU recommendations and in accordance with the European convention for the protection of vertebrate animals used for experimental and other scientific purposes; it was approved by the Ethical Commission of the Medical Faculty in Hradec Králové (Nr. MSMT-56249/2012-310). For the MTD assessment, two or three healthy mice per group were observed for weight loss (the limit was 10%) over 14 days after injection of the solution. For the in vivo activity study, 70 NMRI female mice weighting in the average 31.8 g (SD = 1.27) were fed a standard diet and water ad libitum. A solid Ehrlich tumour was purchased from the Research Institute for Pharmacy and Biochemistry (VUFB) in Prague, and then maintained in NMRI mice by periodical transplantations. The homogenised tumour tissue was inoculated subcutaneously into all mice on day 0, using 0.2 ml of 1/1 (v/v) homogenate freshly prepared in isotonic glucose solution. The tumour-bearing mice were then divided into 5 groups of 14 animals as follows: a control group treated with the pure solvent (DMSO and water), 3 groups of animals treated with compound **1** in doses of 5, 10, and 15 mg/kg i.p. and a positive controls receiving 5 mg/kg cisplatin i.p. (Cisplatin 50 ml/25 mg, EBEWE Pharma, Austria). The solutions were administered on days 1 and 7 in volumes of 0.2 ml per 20 g body weight. On the tenth day, half of the mice were sacrificed, and their tumours were weighed. The remaining animals were left in order to observe their survival.

### Statistical Analysis.

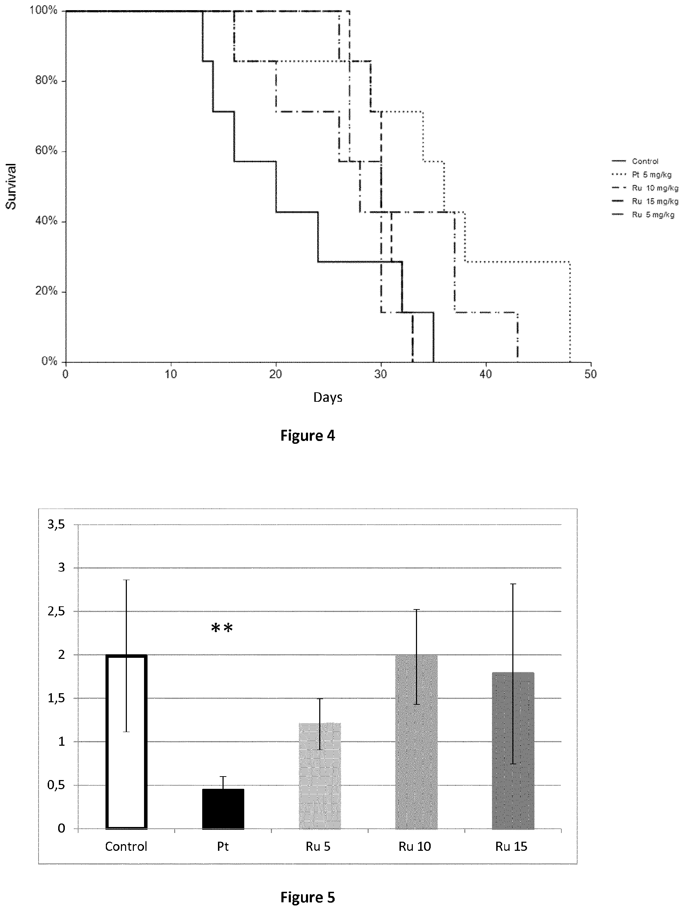

One-Way Analysis of Variance with post-hoc Dunnetts's multiple comparison test was used to detect differences in tumour weight. Kaplan-Meier curves (Figure 4) and logrank tests were used to compare survival times in groups. Here, the level of significance was α=0.05. MS Excel 2003 and NCSS software was used for the calculations and statistical evaluations.

### Results and discussion

During the study of the effect on the survival of tumour-bearing mice, it was observed that the geometric mean of the overall survival of tumour bearing mice without therapy was 20.6 days. Among the three doses of compound **1** tested, only 5 mg/kg prolonged the survival time significantly when compared with untreated tumour-bearing control mice (geom. mean = 31.9 days, *P* = 0.033). 10 mg and 15 mg/kg of compound **1** seemed to exceed the optimal dose, prolonging the survival insignificantly (P > 0.05), with the geometric means of 30.2 and 25.5 days, respectively. The positive control cisplatin was effective too (geom. mean = 33.7 days, P 0.014). An interesting and rare phenomenon was observed in all three groups of compound **1.** Although the tumour was advanced in the later stage of the experiment, all mice treated with compound **1** showed active behaviour, little cachexia and unsuppressed food consumption.

Furthermore, the effect of compound **1** on tumour growth was also investigated. Figure **5** shows the weight of tumours in mice treated with mixture of co-solvent and water, compound **1** at 5, 10 or 15 mg/kg, or cisplatin 5 mg/kg measured on day 10 and documents differences in the effect of the used drugs. Although only cisplatin exhibited a significant inhibitory effect on tumour growth (*P* = 0.0011), there was a slight but insignificant suppression at 5 mg/kg compound 1 (*P* = 0.108). As in the survival study, also here the optimum dose of compound **1** seems to be in the lower part of the range tested.

### References

Urruticoechea, R. Alemany, J. Balart, A. Villanueva, F. Viñals and G. Capellá, Curr. Pharm. Des., 2010, 16, 3-10.
B. Rosenberg, L. Van Camp and T. Krigas, Nature, 1965, 205, 698-699.
B. Rosenberg, L. van Camp, E. B. Grimley and A. J. Thomson, J. Biol. Chem., 1967, 242, 1347-52.
B. Rosenberg, L. Van Camp, J. E. Trosko and V. H. Mansour, Nature, 1969, 222, 385-386.
B. B. Rosenberg, Platin. Met. Rev., 1971, 15, 42-51.
P. Pieter and S. J. Lippard, Encycl. Cancer, 1997, 1, 525-543.
E. Wong and C. M. Giandomenico, Chem. Rev., 1999, 99, 2451-2466.
D. Lebwohl and R. Canetta, Eur. J. Cancer, 1998, 34, 1522-1534.
M. Haga, E. S. Dodsworth, A. B. P. Lever, Inorg. Chem., 1986, 25, 447-453.
I. Brastos, E. Alessio, M. E. Ringenberg and T. B. Rauchfuss, Inorg. Synth., 2010, 35, 148-163.
R. Caspar, C. Cordier, J. B. Waern, C. Guyard-Duhayon, M. Gruselle, P. Le Floch and H. Amouri, Inorg. Chem., 2006, 45, 4071-4078.

## Claims

1. A compound of formula (I): 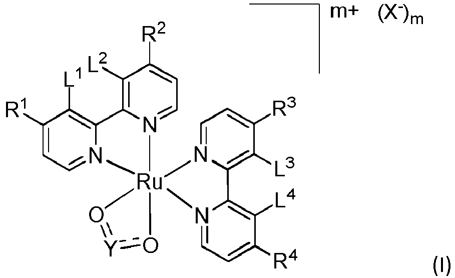 wherein
L¹ and L² are each independently selected in the group consisting of H, halogen, optionally substituted C₁-C₆ alkyl, optionally substituted C₂-C₆ alkenyl, optionally substituted C₂-C₆ alkynyl, optionally substituted carbocycle, optionally substituted aryl, optionally substituted heteroaryl, optionally substituted heterocycle, CN, NO₂, COR¹¹, OR¹² and NR¹³R¹⁴, or
L¹ and L² together represent 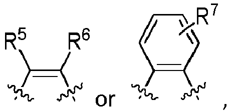
L³ and L⁴ are each independently selected in the group consisting of H, halogen, optionally substituted C₁-C₆ alkyl, optionally substituted C₂-C₆ alkenyl, optionally substituted C₂-C₆ alkynyl, optionally substituted carbocycle, optionally substituted aryl, optionally substituted heteroaryl, optionally substituted heterocycle, CN, NO₂, COR¹⁵, OR¹⁶ and NR¹⁷R¹⁸ or
L³ and L² together represent 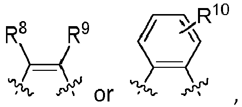
R¹ to R⁶, R⁸ and R⁹ are each independently selected in the group consisting of H, halogen, optionally substituted C₁-C₆ alkyl, optionally substituted C₂-C₆ alkenyl, optionally substituted C₂-C₆ alkynyl, optionally substituted carbocycle, optionally substituted aryl, optionally substituted heteroaryl, optionally substituted heterocycle, CN, NO₂, COR¹⁹, OR²⁰ and NR²¹R²²,
R⁷ and R¹⁰ each independently represent one or several substituent(s) independently selected in the group consisting of H, halogen, optionally substituted C₁-C₆ alkyl, optionally substituted C₂-C₆ alkenyl, optionally substituted C₂-C₆ alkynyl, optionally substituted carbocycle, optionally substituted aryl, optionally substituted heteroaryl, optionally substituted heterocycle, CN, NO₂, COR²³, OR²⁴ and NR²⁵R²⁶,
R¹¹, R¹⁵, R¹⁹ and R²³ are each independently selected in the group consisting of H, optionally substituted C₁-C₆ alkyl, OR²⁷ and NR²⁸R²⁹,
R¹², R¹³, R¹⁴, R¹⁶, R¹⁷, R¹⁸, R²⁰, R²¹, R²², R²⁴, R²⁵, R²⁶, R²⁷, R²⁸ and R²⁹ are each independently selected in the group consisting of H, optionally substituted C₁-C₆ alkyl and optionally substituted CO-C₁-C₆alkyl,
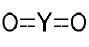 is a bidentate ligand, selected in the group consisting of:
in which 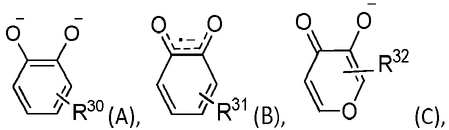
R³⁰, R³¹ and R³² each independently represent one or several substituent(s) independently selected in the group consisting of H, halogen, optionally substituted C₁-C₆ alkyl, CN, NO₂, COR³³, OR³⁴ and NR³⁵R³⁶, R³³ is H, halogen, optionally substituted C₁-C₆ alkyl, OR³⁷ or NR³⁸R³⁹,
R³⁴ to R³⁹ are each independently selected in the group consisting of H, optionally substituted C₁-C₆ alkyl and optionally substituted CO-C₁-C₆alkyl,
X⁻ is a pharmaceutically acceptable anion, preferably selected in the group consisting of PF₆⁻, Cl⁻, Br⁻, BF₄⁻, (C₁-C₆ alkyl)-C(O)O⁻, (C₁-C₆ haloalkyl)-C(O)O⁻, (C₁-C₆ alkyl)-SO₃⁻ and (C₁-C₆ haloalkyl)-SO₃⁻,
m is the global charge of the ruthenium complex of formula: 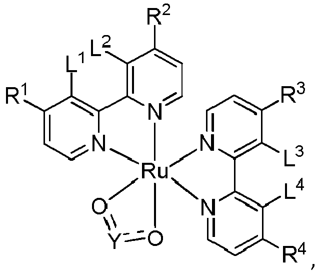 and
m is equal to 0 when 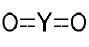 is A and m is equal to +1 when 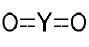 is B or C,
or a pharmaceutically acceptable salt and/or solvate thereof,
with the proviso that the compound of formula (I) or a pharmaceutically acceptable salt and/or solvate thereof is not:
• bis(2,2'-bipyridine)(3,5-bis(1,1-dimethylethyl)-1,2-benzenediolato)ruthenium (II) monohydrate;
• bis(2,2'-bipyridine)(1,2-benzenediolato)ruthenium (II) sesquihydrate;
• bis(2,2'-bipyridine)(3,4,5,6-tetrachloro-1,2-benzenediolato)ruthenium (II) hemihydrate;
• bis(2,2'-bipyridine)(3,5-bis(1,1-dimethylethyl)-3,5-cyclohexadiene-1,2-dionato(radical(1-))ruthenium (II) perchlorate diethyl ether solvate;
• bis(2,2'-bipyridine)(3,5-bis(1,1-dimethylethyl)-3,5-cyclohexadiene-1,2-dionato(radical(1-))ruthenium (II) hexafluorophosphate.

2. The compound according to claim 1, wherein L¹ and L² are H or L¹ and L² together represent 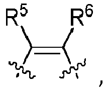 and L³ and L⁴ are H or L³ and L⁴ together represent

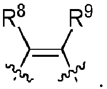

3. The compound according to claim 1 or 2, wherein L¹ and L³ are identical, and L² and L⁴ are identical.

4. The compound according to any one of claims 1 to 3, being a compound of following formula (l-A):

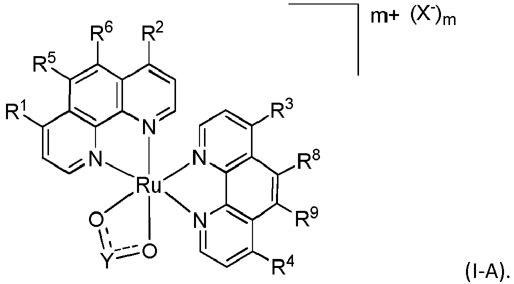

5. The compound according to any one of claims 1 to 4, wherein R¹ to R⁴ are each independently selected in the group consisting of H, halogen and optionally substituted aryl, and preferably are each independently an optionally substituted aryl, notably a phenyl.

6. The compound according to any one of claims 1 to 5, wherein R¹ to R⁴ are identical.

7. The compound according to any one of claims 1 to 6, wherein R⁵, R⁶, R⁸ and R⁹ each independently are H, halogen or OH, preferably H.

8. The compound according to any one of claims 1 to 7, wherein O=Y=O is selected in the group consisting of: 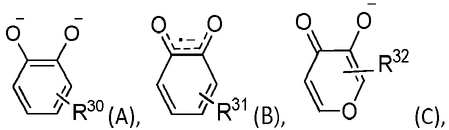 in which R³⁰, R³¹ and R³² each independently represent one or several substituent(s) independently selected from the group consisting of H, halogen, optionally substituted C₁-C₆ alkyl, NO₂ and OR³⁴, preferably selected from H, halogen, C₁-C₆ alkyl, NO₂ and OR³⁴, with R³⁴ being advantageously H or optionally substituted C₁-C₆alkyl, preferably R³⁴ is a C₁-C₆ alkyl such as methyl.

9. The compound according to any one of claims 1 to 8, being selected from the following compounds:

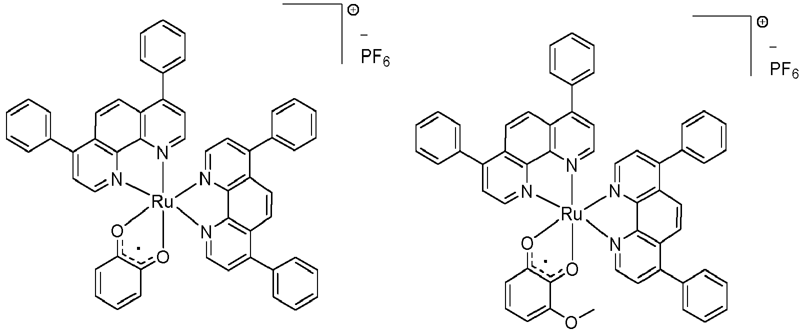

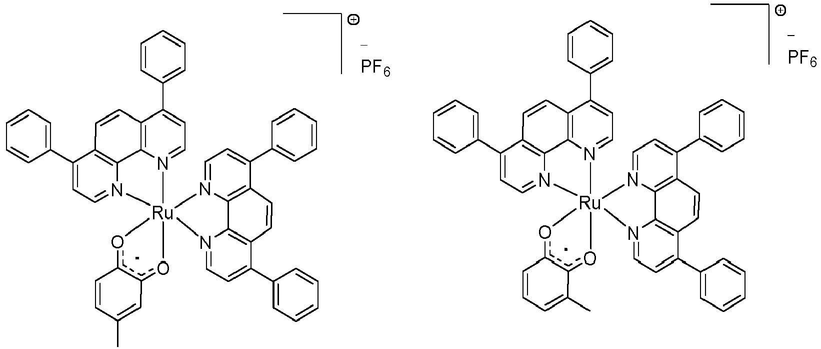

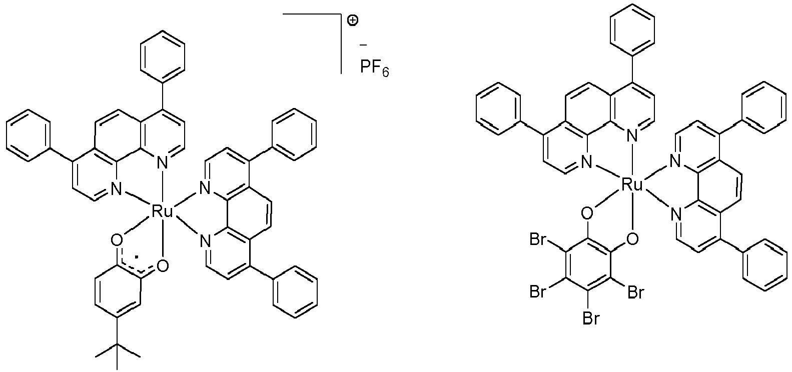

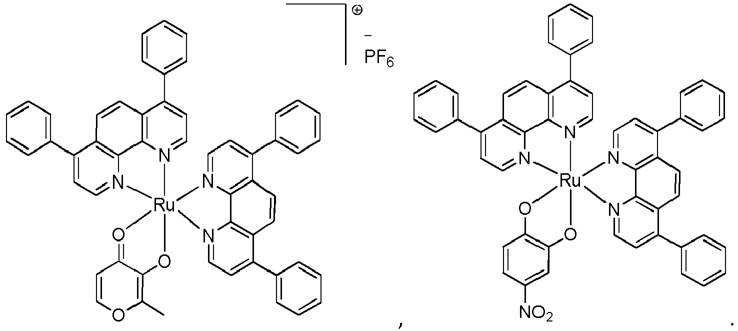

10. A compound according to any one of claims 1 to 9, bis(2,2'-bipyridine)(3,5-bis(1,1-dimethylethyl)-1,2-benzenediolato)ruthenium (II) monohydrate, bis(2,2'-bipyridine)(1,2-benzenediolato)ruthenium (II) sesquihydrate, bis(2,2'-bipyridine)(3,4,5,6-tetrach!oro-1,2-benzenediolato)ruthenium (II) hemihydrate, bis(2,2'-bipyridine)(3,5-bis(1,1-dimethylethyl)-3,5-cyclohexadiene-1,2-dionato(radical(1-))ruthenium (II) perchlorate diethyl ether solvate or bis(2,2'-bipyridine)(3,5-bis(1,1-dimethylethyl)-3,5-cyclohexadiene-1,2-dionato(radical(1-))ruthenium (II) hexafluorophosphate, for use as a drug.

11. A compound according to according to any one of claims 1 to 9, bis(2,2'-bipyridine)(3,5-bis(1,1-dimethylethyl)-1,2-benzenediolato)ruthenium (II) monohydrate, bis(2,2'-bipyridine)(1,2-benzenediolato)ruthenium (II) sesquihydrate, bis(2,2'-bipyridine)(3,4,5,6-tetrachloro-1,2-benzenediolato)ruthenium (II) hemihydrate, bis(2,2'-bipyridine)(3,5-bis(1,1-dimethylethyl)-3,5-cyclohexadiene-1,2-dionato(radical(1-))ruthenium (II) perchlorate diethyl ether solvate or bis(2,2'-bipyridine)(3,5-bis(1,1-dimethylethyl)-3,5-cyclohexadiene-1,2-dionato(radical(1-))ruthenium (II) hexafluorophosphate, for use in the treatment of cancer.

12. The compound for use according to claim 11, wherein cancer is lung cancer, ovarian cancer, colon cancer, colorectal cancer, stomach cancer, testicular cancer, head cancer, neck cancer, breast cancer, kidney cancer, liver cancer, skin cancer, tongue cancer, pancreatic cancer, gall bladder cancer, bladder cancer or leukemia.

13. A pharmaceutical composition comprising at least one pharmaceutically acceptable excipient and at least one active ingredient chosen among a compound according to any one of claims 1 to 9, bis(2,2'-bipyridine)(3,5-bis(1,1-dimethylethyl)-1,2-benzenediolato)ruthenium (II) monohydrate, bis(2,2'-bipyridine)(1,2-benzenediolato)ruthenium (II) sesquihydrate, bis(2,2'-bipyridine)(3,4,5,6-tetrachloro-1,2-benzenediolato)ruthenium (II) hemihydrate, bis(2,2'-bipyridine)(3,5-bis(1,1-dimethylethyl)-3,5-cyclohexadiene-1,2-dionato(radical(1-))ruthenium (II) perchlorate diethyl ether solvate and bis(2,2'-bipyridine)(3,5-bis(1,1-dimethylethyl)-3,5-cyclohexadiene-1,2-dionato(radical(1-))ruthenium (II) hexafluorophosphate,.

14. The pharmaceutical composition according to claim 13 for use in the treatment of cancer, in particular lung cancer, ovarian cancer, colon cancer, colorectal cancer, stomach cancer, testicular cancer, head cancer, neck cancer, breast cancer, kidney cancer, liver cancer, skin cancer, tongue cancer, pancreatic cancer, gall bladder cancer, bladder cancer or leukemia.

15. A method for the preparation of a compound according to any one of claims 1 to 9, bis(2,2'-bipyridine)(3,5-bis(1,1-dimethylethyl)-1,2-benzenediolato)ruthenium (II) monohydrate, bis(2,2'-bipyridine)(1,2-benzenediolato)ruthenium (II) sesquihydrate, bis(2,2'-bipyridine)(3,4,5,6-tetrachloro-1,2-benzenediolato)ruthenium (II) hemihydrate, bis(2,2'-bipyridine)(3,5-bis(1,1-dimethylethyl)-3,5-cyclohexadiene-1,2-dionato(radical(1-))ruthenium (II) perchlorate diethyl ether solvate or bis(2,2'-bipyridine)(3,5-bis(1,1-dimethylethyl)-3,5-cyclohexadiene-1,2-dionato(radical(1-))ruthenium (II) hexafluorophosphate, comprising the following steps:
(i) reacting a compound of formula (II) 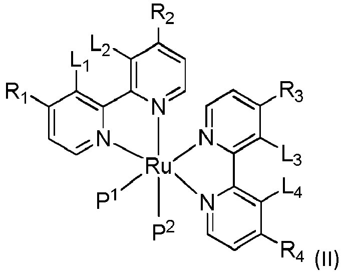
in which L¹ to L⁴ and R¹ to R⁴ are as defined above, and
P¹ and P² each independently represent halogen, OR⁴⁰ or S(O)R⁴¹R⁴²,
R⁴⁰ is H or C₁-C₆ alkyl,
R⁴¹ and R⁴² each independently represent a C₁-C₆ alkyl, in particular a methyl,
preferably P¹ and P² are both halogen, in particular Cl,
with a compound of formula (III) 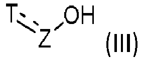 in which 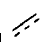 represents a single or a double bond, T is O when 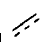 is a double bond and T is OH when 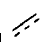 is a single bond and Z is selected in the group consisting of : 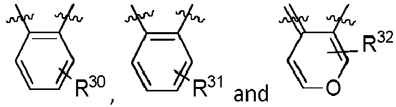 in which R³⁰, R³¹ and R³² are as defined in claim 1. in the presence of a base such as NaOH under inert atmosphere,
(ii) placing the reaction mixture of step (i) in contact with an oxidant such as O₂,
(iii) if necessary, reacting the product resulting from step (ii) with a salt A⁺X⁻, wherein X⁻ is as defined in claim 1 and A⁺ is a counter cation preferably selected from H⁺, Na⁺, K⁺, Li⁺ and N⁺R'R"R"'R"", with R', R", R'" and R"" independently being H or C₁-C₆ alkyl.
